# EUROPEAN PATENT APPLICATION

(11) **EP 4 649 960 A1**
(43) Date of publication of application: **19.11.2025**
(21) Application number: 24749538.5
(22) Date of filing: 18.01.2024
(51) Int. Cl.: A61K 45/00, A61K 31/167, A61K 31/27, A61K 31/137, A61K 31/4704, A61K 31/538, A61K 45/06, A61P 35/00

(54) **USE OF LONG-ACTING ß2AR AGONIST IN PREPARATION OF DRUGS FOR TREATING CANCERS**

(30) Priority: 02.02.2023 CN 202310051916
(71) Applicant: Hong Kong Sxmeterol Pharmaceutical Co., Limited, Kowloon, Hong Kong 999077 (HK)
(72) Inventor: GAO, Hua, Shanghai 200072 (CN); MA, Junxian, Shanghai 200072 (CN)
(74) Representative: karo IP
(86) International application number: PCT/CN2024/072930
(87) International publication number: WO 2024/160057

(57) **Abstract**

Disclosed in the present invention is use of a long-acting β₂AR agonist or pharmaceutically acceptable salts thereof in preparation of medicaments for treating cancer. The long-acting β₂AR agonist of the present invention can target β₂AR on the surface of a cancer cell membrane as a single functional molecular target, and perform targeted inhibition on a self-renewal capability of cancer stem cells to exert a function of inhibiting cancer initiation and progression. Therefore, the β₂AR agonist may be clinically used for treating cancer. In addition, as a repurposed medicament, the long-acting β₂AR agonist may exhibit enhanced clinical translation potential and druggability.

## Description

### TECHNICAL FIELD

The present invention belongs to the field of small molecule medicaments, and in particular to use of a long-acting β₂AR agonist in preparation of medicaments for treating cancer.

### BACKGROUND

Cancer is a leading cause of human death, second only to the top ranked cardiovascular and cerebrovascular diseases in mortality. In 2020, there were 19.3 million new cancer cases and 10 million cancer-related deaths worldwide, accounting for approximately 1/6 of total global deaths that year.

Since the mid-19^{th} century, surgical resection has been officially applied in the treatment of cancer. Subsequently, the discovery of radioactive elements laid a foundation for radiotherapy of cancer. In 1949, the first chemotherapy medicament, nitrogen mustard, was approved by the US FDA for the treatment of Hodgkin's lymphoma. Since then, surgery, radiotherapy, and chemotherapy have gradually developed into three classic methods for the treatment of cancer. In 1953, the discovery of double helix structures of DNA ushered in an era of molecular biology. In recent decades, with in-depth exploration and translational application of cancer at a molecular level, molecular targeted therapy and immunotherapy have emerged as novel treatment methods. Although surgery, radiotherapy, chemotherapy, molecular targeted therapy, and immunotherapy have significantly improved the 5-year survival rate and prognosis of cancer patients, the 5-year survival rate of most cancers, such as lung cancer, liver cancer, pancreatic cancer, and gastric cancer, is still less than 30%. Therefore, there is an urgent need to develop a new medicament with good therapeutic effects.

Small molecule targeted medicaments have advantages such as convenience in synthesis, low cost, fast absorption, and easy storage. Imatinib is the first small molecule targeted medicament used in clinical practice, which can specifically inhibit activation of BCR⁻ABL tyrosine kinase, a fusion protein formed by proto-oncogene recombination, and induce cancer cell apoptosis. Clinical data shows that as a first-line treatment for chronic myeloid leukemia, patients treated by imatinib have an overall 10-year survival rate of 83.3%. By the end of 2020, there have been 89 small molecule targeted anti-cancer medicaments approved by the US FDA and China's National Medical Products Administration, such as Gefitinib (an inhibitor targeting an epidermal growth factor receptor-tyrosine kinase (EGFR-TK)), Everolimus (an inhibitor targeting mTOR), and Vemurafenib (an inhibitor targeting BRAF), all of which are classic clinical medicaments in cancer treatment.

However, for the small molecule targeted medicaments, unstable response, medicament resistance, recurrence, and other phenomena are still main problems faced by clinical cancer patients after medication. In recent years, many studies have demonstrated that cancer tissues have heterogeneity, and even in the same cancer tissue, there are cancer cells with completely different genotypes, phenotypes, and functions.

In 1937, Furth and Kanh discovered that transplanting an individual mouse cancer cell into a recipient mouse could form a new cancer. In 1960, Pierce discovered highly tumorigenic cells in malignant teratoma, and the highly tumorigenic cells could further differentiate into a variety of non-tumorigenic cells. In the early 1990s, with research of hematopoietic stem cells and development of a flow cytometry technology, Dick et al. discovered in a mouse transplant model of acute myeloid leukemia (AML) that only CD34⁺CD38⁻ cancer cells could form transplanted cancers, and the initiating cell frequency of the transplanted cancers was one in a million. This group of cells are defined as cancer stem cells in AML, which is the first time that the concept of "cancer stem cells" has been explicitly proposed. In 2003, Clarke first applied the concept of "cancer stem cells" and a xenotransplantation experimental method to solid breast cancers, and determined that CD44⁺CD24⁻ breast cancer cells are tumorigenic. In the following 20 years, exploration of cancer stem cells continued to deepen and was confirmed in a variety of types of cancer.

A large number of animal experimental results further indicate that cancer stem cells account for a relatively small proportion of an entire cancer cell population, possess self-renewal capability and a potential for multicellular differentiation, and are the main causes of initiation, growth, medicament resistance, immune escape, recurrence, and metastasis of cancers. A hypothesis is proposed that targeting and inhibiting cancer stem cells is the most ideal approach of cancer treatment in clinical practice.

However, there is no available single molecule targeted medicament that specifically targets cancer stem cells, for cancer treatment in clinical practice. Therefore, searching for specific cancer stem cell targets and developing single molecule targeted medicaments that specifically target cancer stem cells and have strong efficacy, high safety, and low cost are critical. This may greatly prolong and improve the survival of cancer patients.

### SUMMARY

The present invention is directed to solve at least one of the technical problems in the prior art. Therefore, the present invention provides use of a long-acting β₂AR agonist or pharmaceutically acceptable salts thereof in preparation of medicaments for treating cancer, where the long-acting β₂AR agonist can target and inhibit cancer stem cells for cancer treatment.

According to one aspect of the present invention, use of a long-acting β₂AR agonist or pharmaceutically acceptable salts thereof in preparation of medicaments for treating cancer is provided.

According to a specific embodiment of the present invention, the present invention has at least the following beneficial effects: The specific long-acting β₂AR agonist targets β₂AR on the surface of a cancer cell membrane as a single functional molecular target, and perform targeted inhibition on a self-renewal capability of cancer stem cells to exert a function of inhibiting cancer initiation and progression. Therefore, the long-acting β₂AR agonist may be clinically used for treating cancer. In addition, as a repurposed medicament, the long-acting β₂AR agonist may exhibit enhanced clinical translation potential and druggability.

In some embodiments of the present invention, the long-acting β₂AR agonist includes all clinically recognized long-acting specific β₂ adrenoreceptor agonists.

In some embodiments of the present invention, the long-acting β₂AR agonist is a specific β₂ adrenoceptor agonist with a duration of efficacy of not shorter than 12 h.

In some embodiments of the present invention, the long-acting β₂AR agonist includes at least one of arformoterol, bambuterol, clenbuterol, formoterol, indacaterol, olodaterol, salmeterol, tulobuterol, and vilanterol.

In some preferable embodiments of the present invention, the long-acting β₂AR agonist includes at least one of arformoterol tartrate, bambuterol hydrochloride, clenbuterol hydrochloride, formoterol hemifumarate, indacaterol maleate, olodaterol hydrochloride, salmeterol xinafoate, tulobuterol hydrochloride, and vilanterol trifenate.

In some embodiments of the present invention, the cancer includes at least one of breast cancer, colorectal cancer, lung cancer, head and neck cancer, esophageal cancer, bladder cancer, melanoma, hematoma, prostate cancer, liver cancer, ovarian cancer, kidney cancer, gastric cancer, pancreatic cancer, brain cancer, osteosarcoma, and cervical cancer.

In some embodiments of the present invention, the medicaments for treating cancer are medicaments that target and inhibit cancer stem cells.

In some preferable embodiments of the present invention, the targeting and inhibiting cancer stem cells refers to targeting and binding an extracellular region of a β₂AR of a cancer stem cell.

In some embodiments of the present invention, a dosage form of the medicaments includes aerosol, spray, emulsion, suspension, enema, paste, ointment, gel, lotion, suppository, patch, film, capsule, tablet, pill, powder, granule, tincture, injection, syrup, or solution.

In some embodiments of the present invention, an administration route of the medicaments includes oral administration, sublingual administration, inhalation administration, mucosal administration, intravenous injection, arterial injection, intramuscular injection, intradermal injection, subcutaneous injection, intraperitoneal injection, rectal administration, vaginal administration, transdermal administration, or local administration. Here, the medicaments of the present invention may be administered/applied via any suitable route, for example, oral administration in the form of capsules, injection administration, local application in the form of ointments or lotions, rectal administration in the form of suppositories, or transdermal administration in the form of a patch delivery system.

In some embodiments of the present invention, the pharmaceutically acceptable salts are salts formed by the long-acting β₂AR agonist and an acid or a base; the acid includes an inorganic acid or an organic acid; the inorganic acid is selected from hydrochloric acid, hydrobromic acid, hydroiodic acid, nitric acid, phosphoric acid, sulfuric acid, or perchloric acid; the organic acid includes acetic acid, oxalic acid, malic acid, maleic acid, lactic acid, pyruvic acid, methanesulfonic acid, ethanesulfonic acid, p-toluenesulfonic acid, salicylic acid, tartaric acid, benzenesulfonic acid, benzoic acid, camphorsulfonic acid, citric acid, succinic acid, fumaric acid, phenylacetic acid, triphenylacetic acid, mandelic acid, gluconic acid, glutamic acid, isethionic acid, mucic acid, pamoic acid, pantothenic acid, xinafoic acid, or malonic acid; and the base is an inorganic base containing an alkaline metal cation, alkaline-earth metal cation, or ammonium cation salt.

In some embodiments of the present invention, the medicaments further include a pharmaceutically acceptable carrier.

Here, the term "pharmaceutically acceptable carrier" refers to a pharmaceutically acceptable material, composition, or carrier, such as a liquid or solid filler, stabilizer, dispersant, suspending agent, diluent, excipient, thickener, solvent, or encapsulating material, involved in carrying or transporting a useful compound of the present invention within or to a patient's body to perform an expected function of the useful compound. Typically, such a construct may be carried to or transported from one organ or part of the body to another. Some examples of materials that may be used as the pharmaceutically acceptable carrier include: sugar, such as lactose, glucose, and sucrose; starch, such as corn starch and potato starch; cellulose and derivatives thereof, such as sodium carboxymethyl cellulose, ethyl cellulose, and cellulose acetate; powdered tragacanth gum; malt; gelatin; talc; excipient, such as cocoa butter and suppository wax; oil, such as peanut oil, cottonseed oil, safflower oil, sesame oil, olive oil, corn oil, and soybean oil; diol, such as propylene glycol; polylol, such as glycerol, sorbitol, mannitol, and polyethylene glycol; esters, such as ethyl oleate and ethyl laurate; agar; buffering agent, such as magnesium hydroxide and aluminum hydroxide; surfactant; alginate; pyrogen-free water; isotonic saline water; Ringer's solution; ethanol; phosphate buffer solution; and other non-toxic compatible substances used in pharmaceutical preparations. As used herein, the "pharmaceutically acceptable carrier" further includes any and all coatings, antibacterial and antifungal agents, absorption delaying agents, and the like that are compatible with the activity of the compound of the present invention and physiologically acceptable to patients. A supplementary active compound may also be incorporated into a composition.

### BRIEF DESCRIPTION OF THE DRAWINGS

The present invention is further described in conjunction with the accompanying drawings and examples. In the figures,
FIG. 1 shows mechanism of action of long-acting β₂AR agonist on cancers in examples of the present invention;
FIG. 2 is a schematic diagram of expression of β₂AR in a variety of types of cancer tissues in examples of the present invention;
FIG. 3 is a flowchart of an animal experiment in Example 1 of the present invention;
FIG. 4 shows an animal experimental result of salmeterol xinafoate inhibiting orthotopic breast cancer in an example of the present invention;
FIG. 5 shows an animal experimental result of salmeterol xinafoate inhibiting colorectal cancer in an example of the present invention;
FIG. 6 shows an animal experimental result of salmeterol xinafoate inhibiting lung cancer in an example of the present invention;
FIG. 7 shows an animal experimental result of salmeterol xinafoate inhibiting head and neck cancer in an example of the present invention;
FIG. 8 shows an animal experimental result of salmeterol xinafoate inhibiting esophageal cancer in an example of the present invention;
FIG. 9 shows an animal experimental result of salmeterol xinafoate inhibiting bladder cancer in an example of the present invention;
FIG. 10 shows an animal experimental result of salmeterol xinafoate inhibiting melanoma in an example of the present invention;
FIG. 11 shows experimental results of salmeterol xinafoate inhibiting tumor sphere formation of breast cancer cells in an example of the present invention;
FIG. 12 shows experimental results of salmeterol xinafoate inhibiting tumor sphere formation of colorectal cancer cells in an example of the present invention;
FIG. 13 shows experimental results of salmeterol xinafoate inhibiting tumor sphere formation of lung cancer cells in an example of the present invention;
FIG. 14 shows experimental results of salmeterol xinafoate inhibiting tumor sphere formation of head and neck cancer cells in an example of the present invention;
FIG. 15 shows experimental results of salmeterol xinafoate inhibiting tumor sphere formation of esophageal cancer cells in an example of the present invention;
FIG. 16 shows experimental results of salmeterol xinafoate inhibiting tumor sphere formation of bladder cancer cells in an example of the present invention;
FIG. 17 shows experimental results of salmeterol xinafoate inhibiting tumor sphere formation of melanoma cells in an example of the present invention;
FIG. 18 shows experimental results of salmeterol xinafoate inhibiting tumor sphere formation of hematoma cells in an example of the present invention;
FIG. 19 shows experimental results of salmeterol xinafoate inhibiting tumor sphere formation of prostate cancer cells in an example of the present invention;
FIG. 20 shows experimental results of salmeterol xinafoate inhibiting tumor sphere formation of liver cancer cells in an example of the present invention;
FIG. 21 shows experimental results of salmeterol xinafoate inhibiting tumor sphere formation of ovarian cancer cells in an example of the present invention;
FIG. 22 shows experimental results of salmeterol xinafoate inhibiting tumor sphere formation of kidney cancer cells in an example of the present invention;
FIG. 23 shows experimental results of salmeterol xinafoate inhibiting tumor sphere formation of gastric cancer cells in an example of the present invention;
FIG. 24 shows experimental results of salmeterol xinafoate inhibiting tumor sphere formation of pancreatic cancer cells in an example of the present invention;
FIG. 25 shows experimental results of salmeterol xinafoate inhibiting tumor sphere formation of brain cancer cells in an example of the present invention;
FIG. 26 shows experimental results of salmeterol xinafoate inhibiting tumor sphere formation of osteosarcoma cells in an example of the present invention;
FIG. 27 shows experimental results of salmeterol xinafoate inhibiting tumor sphere formation of cervical cancer cells in an example of the present invention;
FIG. 28 shows experimental results of arformoterol tartrate inhibiting tumor sphere formation of a variety of cancer cells in an example of the present invention;
FIG. 29 shows experimental results of bambuterol hydrochloride inhibiting tumor sphere formation of a variety of cancer cells in an example of the present invention;
FIG. 30 shows experimental results of clenbuterol hydrochloride inhibiting tumor sphere formation of a variety of cancer cells in an example of the present invention;
FIG. 31 shows experimental results of formoterol hemifumarate inhibiting tumor sphere formation of a variety of cancer cells in an example of the present invention;
FIG. 32 shows experimental results of indacaterol maleate inhibiting tumor sphere formation of a variety of cancer cells in an example of the present invention;
FIG. 33 shows experimental results of olodaterol hydrochloride inhibiting tumor sphere formation of a variety of cancer cells in an example of the present invention;
FIG. 34 shows experimental results of tulobuterol hydrochloride inhibiting tumor sphere formation of a variety of cancer cells in an example of the present invention; and
FIG. 35 shows experimental results of vilanterol trifenate inhibiting tumor sphere formation of a variety of cancer cells in an example of the present invention.

### DETAILED DESCRIPTION

The following provides a clear and complete description of the concept and technical effects of the present invention in conjunction with examples, to fully understand the objectives, features, and effects of the present invention. It is apparent that the described examples are only some, but not all examples of the present invention. Based on the examples of the present invention, other examples obtained by those skilled in the art without creative labor are within the protection scope of the present invention. The experimental methods used in the examples are conventional methods unless otherwise specified, and the materials, reagents, and the like used may be obtained from commercial sources unless otherwise specified.

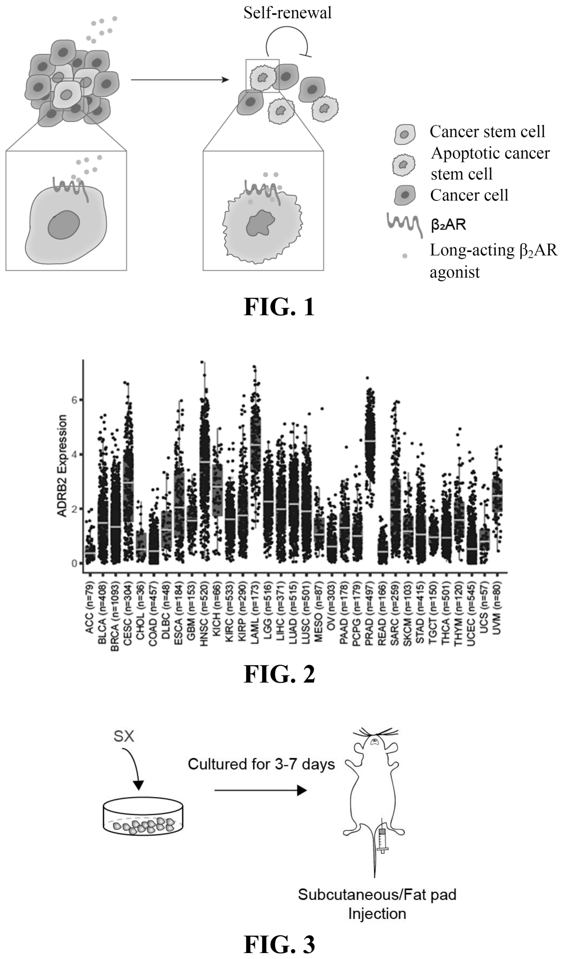

In the examples of the present invention, molecular targeted medicaments were developed targeting a single functional molecular target specifically expressed on the surface of cancer stem cells. It was found that long-acting β₂AR agonist can target and inhibit cancer stem cells for treating cancer. The mechanism of action is shown in FIG. 1.

The adrenoceptor beta 2 (β₂AR) is a member of the G-protein coupled receptor superfamily, which is the first discovered and extensively studied. The β₂AR is expressed on the cell membrane and has a classic seven-transmembrane structure. The long-acting β₂AR agonist is commonly used as bronchodilators to treat asthma and chronic obstructive pulmonary disease. The duration of efficacy of the long-acting β₂AR agonist is not less than 12 h, and the duration of action is long. The long-acting β₂AR agonist in the present invention include all clinically recognized long-acting specific β₂ adrenoreceptor agonists. A total of 9 long-acting β₂AR agonists are used in the examples of the present invention, including: arformoterol tartrate, bambuterol hydrochloride, clenbuterol hydrochloride, formoterol hemifumarate, indacaterol maleate, olodaterol hydrochloride, salmeterol xinafoate, tulobuterol hydrochloride, and vilanterol trifenate, all of which are small molecule medicaments that specifically target β₂AR.

An analysis of a genomic expression profile of clinical cancer patients demonstrates that β₂AR adrenoreceptors are expressed in a variety of cancer tissues (FIG. 2). The examples of the present invention have found and demonstrated that use of any of specific long-acting β₂AR agonists alone can inhibit the function of cancer stem cells for treating cancer. Namely, the β₂AR adrenoceptor can serve as a single functional molecular target specific to cancer stem cells, and the specific long-acting β₂AR agonists are small molecule targeted medicaments to the single functional molecular target.

The mRNA expression of ADRB2 (β₂AR) in a variety of types of cancer tissues (data from The Cancer Genome Atlas database). ACC, Adrenal Cancer; BLCA, Bladder Cancer; BRCA, Breast Cancer; CESC, Cervical Cancer; CHOL, Bile Duct Cancer; COAD, Colon Cancer; DLBC, Large B-cell Lymphoma; ESCA, Esophageal Cancer; GBM, Glioma; HNSC, Head and Neck Cancer; KICH, Renal Chromophobe Cell Carcinoma; KIRC, Renal Clear Cell Carcinoma; KIRP, Papillary Renal Cell Carcinoma; LAML, Myeloid Leukemia; LGG, Low Grade Glioma of the Brain; LIHC, Liver Cancer; LUAD, Lung Adenocarcinoma; LUSC, Lung Squamous Cell Carcinoma; MESO, Mesothelioma; OV, Ovarian Cancer; PAAD, Pancreatic Cancer; PCPG, Pheochromocytoma and Paraganglioma; PRAD, Prostate Cancer; READ, Rectal Cancer; SARC, Sarcoma; SKCM, Melanoma; STAD, Gastric Cancer; TGCT, Testicular Cancer; THCA, Thyroid Cancer; THYM, Thymic Carcinoma; UCEC, Endometrial Cancer; UCS, Uterine Sarcoma; and UVM, Uveal Melanoma.

Since salmeterol xinafoate has low price and easy availability, and is a long-acting β₂AR agonist that is the earliest used in clinical practice and has the largest market share, the examples of the present invention first conduct animal experiments using salmeterol xinafoate as an example.

### Example 1: Therapeutic effects of salmeterol xinafoate on cancer

### 1.1 Animal experiments of salmeterol xinafoate inhibiting orthotopic cancer

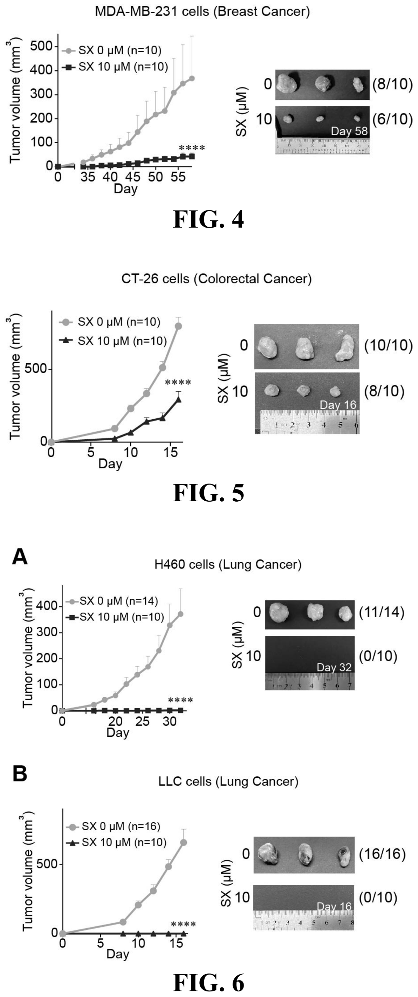

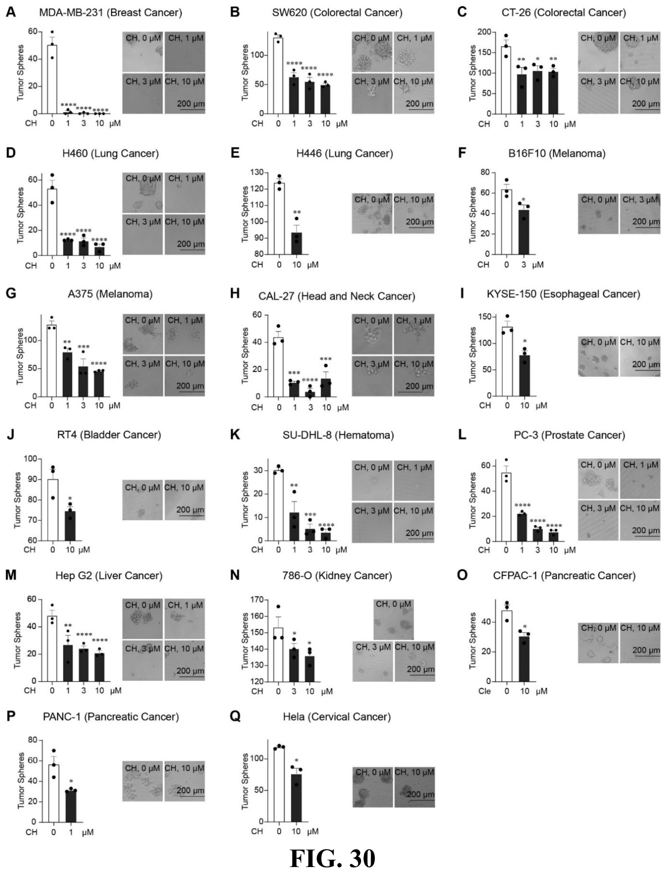

Experimental method and steps: Cancer cells were treated with salmeterol xinafoate (SX) in vitro for 3-7 d, and a subcutaneous or orthotopic tumorigenesis experiment was conducted on mice. The procedure of the animal experiment is shown in FIG. 3. The results of this example show that the salmeterol xinafoate has inhibiting effects on initiation and progression of breast cancer, colorectal cancer, lung cancer, head and neck cancer, esophageal cancer, bladder cancer, and melanoma (FIG. 4-FIG. 10).

### 1.1.1 Salmeterol xinafoate inhibits initiation and progression of orthotopic breast cancer

MDA-MB-231 breast cancer cells were treated with 10 µM salmeterol xinafoate (SX) for 7 d, and cells (1×10⁴/mouse) were injected into the orthotopic mammary fat pads on both sides of the body of mice in a control group and an SX treated group (5 mice/group) to conduct a tumorigenesis experiment. 8 orthotopic cancers formed at 10 injection sites in the orthotopic mammary fat pads of 5 mice in the control group, and only 6 orthotopic cancers formed at 10 injection sites in the orthotopic mammary fat pads of 5 mice in the SX treated group.

Experimental results in FIG. 4 show cancer growth curves and representative cancer photos. Using a Two-way ANOVA analysis method and Fisher's LSD test as a post test, statistical differences in the cancer growth curves were analyzed (****, *P*<0.0001)*.* The experimental results demonstrate that the salmeterol xinafoate has therapeutic effects on breast cancer and inhibits the initiation and progression of orthotopic breast cancer.

### 1.1.2 Salmeterol xinafoate inhibits initiation and progression of subcutaneous colorectal cancer

CT-26 colorectal cancer cells were treated with 10 µM salmeterol xinafoate (SX) for 7 d, and cells (1×10⁵/mouse) were subcutaneously injected on both sides of the back of mice in a control group and an SX treated group (5 mice/group) to conduct a tumorigenesis experiment. 10 cancers formed subcutaneously at 10 injection sites of 5 mice in the control group, and 8 cancers formed subcutaneously at 10 injection sites of 5 mice in the SX treated group.

Experimental results in FIG. 5 show cancer growth curves and representative cancer photos. Using a Two-way ANOVA analysis method and Fisher's LSD test as a post test, statistical differences in the cancer growth curves were analyzed (****, *P*<0.0001)*.* The experimental results demonstrate that the salmeterol xinafoate has therapeutic effects on colorectal cancer and inhibits the initiation and progression of subcutaneous colorectal cancer.

### 1.1.3 Salmeterol xinafoate inhibits initiation and progression of subcutaneous lung cancer

In FIG. 6A, H460 lung cancer cells were treated with 10 µM salmeterol xinafoate (SX) for 7 d, and cells (3×10⁵/mouse) were subcutaneously injected on both sides of the back of mice in a control group (7 mice) and an SX treated group (5 mice) to conduct a tumorigenesis experiment. 11 cancers formed subcutaneously at 14 injection sites of 7 mice in the control group, while no cancers formed subcutaneously at 10 injection sites of 5 mice in the SX treated group.

In FIG. 6B, LLC lung cancer cells were treated with 10 µM salmeterol xinafoate (SX) for 7 d, and cells (1×10⁵/mouse) were subcutaneously injected on both sides of the back of mice in a control group (8 mice) and an SX treated group (5 mice) to conduct a tumorigenesis experiment. Cancers formed subcutaneously at all 16 injection sites of 8 mice in the control group, while no cancers formed subcutaneously at 10 injection sites of 5 mice in the SX treated group.

Experimental results in FIG. 6 show cancer growth curves and representative cancer photos. Using a Two-way ANOVA analysis method and Fisher's LSD test as a post test, statistical differences in the cancer growth curves were analyzed (****, *P*<0.0001)*.* The experimental results demonstrate that the salmeterol xinafoate has therapeutic effects on lung cancer and inhibits the initiation and progression of subcutaneous lung cancer.

### 1.1.4 Salmeterol xinafoate inhibits initiation and progression of subcutaneous head and neck cancer

CAL-27 head and neck cancer cells were treated with 10 µM salmeterol xinafoate (SX) for 3 d, and cells (1×10⁶/mouse) were subcutaneously injected on both sides of the back of mice in a control group and an SX treated group (3 mice/group) to conduct a tumorigenesis experiment.

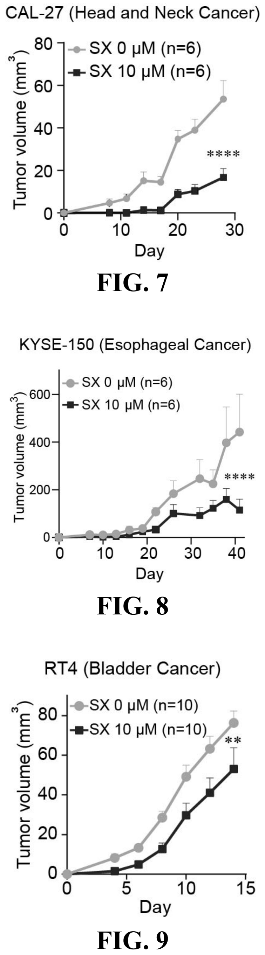

Experimental results in FIG. 7 show cancer growth curves. Using a Two-way ANOVA analysis method and Fisher's LSD test as a post test, statistical differences in the cancer growth curves were analyzed (****, *P*<0.0001)*.* The experimental results demonstrate that the salmeterol xinafoate has therapeutic effects on head and neck cancer and inhibits the initiation and progression of subcutaneous head and neck cancer.

### 1.1.5 Salmeterol xinafoate inhibits initiation and progression of subcutaneous esophageal cancer

KYSE-150 esophageal cancer cells were treated with 10 µM salmeterol xinafoate (SX) for 3 d, and cells (1×10⁶/mouse) were subcutaneously injected on both sides of the back of mice in a control group and an SX treated group (3 mice/group) to conduct a tumorigenesis experiment.

Experimental results in FIG. 8 show cancer growth curves. Using a Two-way ANOVA analysis method and Fisher's LSD test as a post test, statistical differences in the cancer growth curves were analyzed (****, *P*<0.0001)*.* The experimental results demonstrate that the salmeterol xinafoate has therapeutic effects on esophageal cancer and inhibits the initiation and progression of subcutaneous esophageal cancer.

### 1.1.6 Salmeterol xinafoate inhibits initiation and progression of subcutaneous bladder cancer

RT4 bladder cancer cells were treated with 10 µM salmeterol xinafoate (SX) for 3 d, and cells (1×10⁶/mouse) were subcutaneously injected on both sides of the back of mice in a control group and an SX treated group (5 mice/group) to conduct a tumorigenesis experiment.

Experimental results in FIG. 9 show cancer growth curves. Using a Two-way ANOVA analysis method and Fisher's LSD test as a post test, statistical differences in the cancer growth curves were analyzed (****, *P*<0.0001)*.* The experimental results demonstrate that the salmeterol xinafoate has therapeutic effects on bladder cancer and inhibits the initiation and progression of subcutaneous bladder cancer.

### 1.1.7 Salmeterol xinafoate inhibits initiation and progression of orthotopic melanoma

B16F10 melanoma cells were treated with 10 µM salmeterol xinafoate (SX) for 3 d, and cells (1×10⁶/mouse) were subcutaneously injected on both sides of the back of mice in a control group and an SX treated group (4 mice/group) to conduct a tumorigenesis experiment. Cancers formed subcutaneously at all 8 injection sites of 4 mice in the control group, and cancers formed subcutaneously also at all 8 injection sites of 4 mice in the SX treated group.

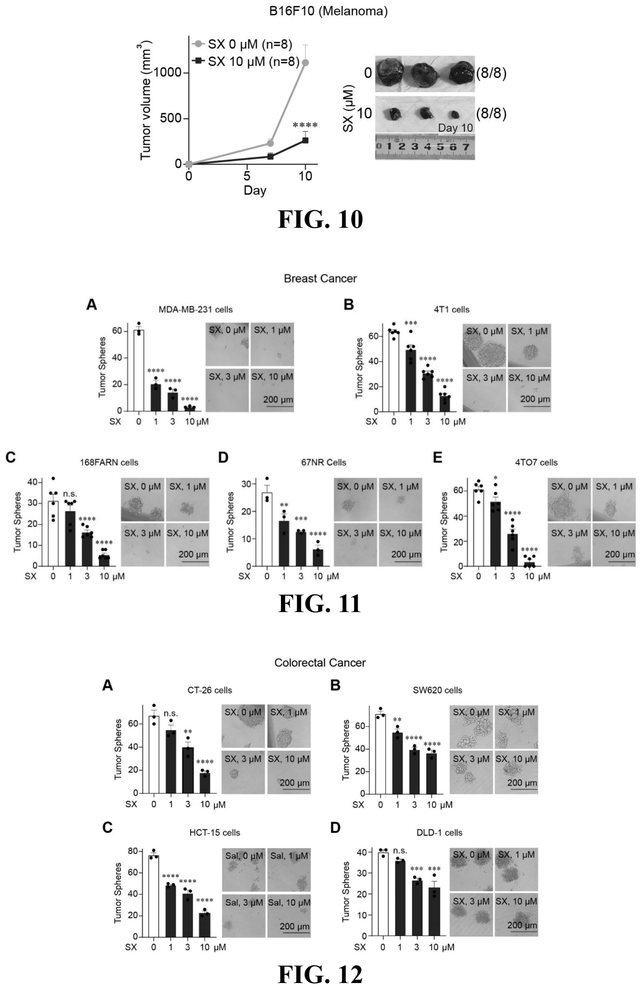

Experimental results in FIG. 10 show cancer growth curves and representative cancer photos. Using a Two-way ANOVA analysis method and Fisher's LSD test as a post test, statistical differences in the cancer growth curves were analyzed (****, *P*<0.0001)*.* The experimental results demonstrate that the salmeterol xinafoate has therapeutic effects on melanoma and inhibits the initiation and progression of orthotopic melanoma.

The above in vivo tumorigenesis experiments demonstrate that the specific long-acting β₂AR agonist, salmeterol xinafoate, has therapeutic effects on a variety of cancers, not only inhibiting cancer initiation but also significantly inhibiting cancer growth.

### 1.2 Experiments of salmeterol xinafoate inhibiting tumor sphere formation

Numerous studies have demonstrated that cancer stem cells have self-renewal capability, which determines the initiation and progression of cancers. As a gold standard for detecting the self-renewal capability of cancer stem cells in vitro, a tumor sphere formation assay may detect individual cancer stem cell that continuously self-renew in a conditioned medium and form tumor spheres consisting of multiple cells. This in vitro experiment is often used to test the responsiveness of clinical patients to anticancer medicaments, and guide in vivo medication of the clinical patients.

Also, via the tumor sphere formation assays, this example also demonstrated that salmeterol xinafoate can inhibit the self-renewal capability of the cancer stem cells derived from the seven cancer types mentioned above (FIG. 11-FIG. 17).

### 1.2.1 Salmeterol xinafoate inhibits tumor sphere formation capability of breast cancer cells

According to culture conditions in Table 1, breast cancer cells MDA-MB-231 (A), 4T1 (B), 168FARN (C), 67NR (D), and 4TO7 (E) were subjected to tumor sphere formation assays, and treated with salmeterol xinafoate (SX) at concentrations of 1 µM, 3 µM, or 10 µM, respectively. After 7 d, tumor spheres in the control group and the SX treated group were counted and photographed, respectively.

**Table 1 Culture conditions for tumor sphere formation assay of breast cancer cells**

| Cells | Cell count | Culture conditions |
|---|---|---|
| MDA-MB-231 | 1000 | MEBM, containing 1×B27, EGF (20 ng/ml), bFGF (20 ng/ml), heparin (4 µg/ml), insulin (5 µg/ml), hydrocortisone (0.5 µg/ml), 1×NEAA, penicillin/streptomycin (100 U/ml/0.1 mg/ml) |
| 4T1 | 1000 | DMEM/F12, containing 1 ×B27, EGF (20 ng/ml), bFGF (20 ng/ml), insulin (10 µg/ml), hydrocortisone (20 µg/ml), 1×NEAA, penicillin/streptomycin (100 U/ml/0.1 mg/ml) |
| 168FARN | 1000 | DMEM/F12, containing 1 ×B27, EGF (20 ng/ml), bFGF (20 ng/ml), insulin (10 µg/ml), hydrocortisone (20 µg/ml), 1 ×NEAA, penicillin/streptomycin (100 U/ml/0.1 mg/ml) |
| 67NR | 1000 | DMEM/F12, containing 1 ×B27, EGF (20 ng/ml), bFGF (20 ng/ml), insulin (10 µg/ml), hydrocortisone (20 µg/ml), 1 ×NEAA, penicillin/streptomycin (100 U/ml/0.1 mg/ml) |
| 4TO7 | 1000 | DMEM/F12, containing 1×B27, EGF (10 ng/ml), bFGF (10 ng/ml), heparin (4 µg/ml), insulin (5 µg/ml), hydrocortisone (20 µg/ml), 1×NEAA, penicillin/streptomycin (100 U/ml/0.1 mg/ml) |

Experimental results in FIG. 11 show the number of tumor spheres from at least 3 independent experiments and representative tumor sphere photos. Using a One-way ANOVA analysis method and Fisher's LSD test as a post test, statistical differences in the number of tumor spheres in all the groups were analyzed (*, *P*<0.05; **, *P*<0.01; ***, *P*<0.001; ****, *P*<0.0001). The experimental results demonstrate that the salmeterol xinafoate inhibited the self-renewal capability of the breast cancer stem cells in a concentration dependent manner.

### 1.2.2 Salmeterol xinafoate inhibits tumor sphere formation capability of colorectal cancer cells

According to culture conditions in Table 2, colorectal cancer cells CT-26 (A), SW620 (B), HCT-15 (C), and DLD-1 (D) were subjected to tumor sphere formation assays, and treated with salmeterol xinafoate (SX) at concentrations of 1 µM, 3 µM, or 10 µM, respectively. After 7 d, tumor spheres in the control group and the SX treated group were counted and photographed, respectively.

**Table 2 Culture conditions for tumor sphere formation assay of colorectal cancer cells**

| Cells | Cell count | Culture conditions |
|---|---|---|
| CT-26 | 1000 | DMEM/F12, containing 1 ×B27, EGF (20 ng/ml), bFGF (20 ng/ml), insulin (5 µg/ml), BSA (0.4%), 1 ×NEAA, penicillin/streptomycin (100 U/ml/0.1 mg/ml) |
| SW620 | 1000 | DMEM/F12, containing 1×B27, EGF (20 ng/ml), bFGF (10 ng/ml), insulin (5 µg/ml), BSA (0.4%), 1 ×NEAA, penicillin/streptomycin (100 U/ml/0.1 mg/ml) |
| HCT-15 | 1000 | DMEM/F12, containing 1 ×B27, EGF (20 ng/ml), bFGF (20 ng/ml), insulin (5 µg/ml), BSA (0.4%), 1 ×NEAA, penicillin/streptomycin (100 U/ml/0.1 mg/ml) |
| DLD-1 | 1000 | DMEM/F12, containing 1 ×B27, EGF (20 ng/ml), bFGF (20 ng/ml), insulin (5 µg/ml), BSA (0.4%), 1 ×NEAA, penicillin/streptomycin (100 U/ml/0.1 mg/ml) |

Experimental results in FIG. 12 show the number of tumor spheres from 3 independent experiments and representative tumor sphere photos. Using a One-way ANOVA analysis method and Fisher's LSD test as a post test, statistical differences in the number of tumor spheres in all the groups were analyzed (**, *P*<0.01*;* ***, *P*<0.001; ****, *P*<0.0001; n.s., no significant difference). The experimental results demonstrate that the salmeterol xinafoate inhibited the self-renewal capability of the colorectal cancer stem cells in a concentration dependent manner.

### 1.2.3 Salmeterol xinafoate inhibits tumor sphere formation capability of lung cancer cells

According to culture conditions in Table 3, lung cancer cells LLC (A), H1299 (B), H460 (C), and H446 (D) were subjected to tumor sphere formation assays, and treated with salmeterol xinafoate (SX) at concentrations of 1 µM, 3 µM, or 10 µM, respectively. After 7 d, tumor spheres in the control group and the SX treated group were counted and photographed, respectively.

**Table 3 Culture conditions for tumor sphere formation assay of lung cancer cells**

| Cells | Cell count | Culture conditions |
|---|---|---|
| H460 | 1000 | DMEM/F12, containing 1×B27, EGF (20 ng/ml), bFGF (10 ng/ml), insulin (2 µg/ml), 1×NEAA, penicillin/streptomycin (100 U/ml/0.1 mg/ml) |
| H1299 | 1000 | DMEM/F12, containing 1×B27, EGF (20 ng/ml), bFGF (10 ng/ml), insulin (2 µg/ml), 1×NEAA, penicillin/streptomycin (100 U/ml/0.1 mg/ml) |
| LLC | 1000 | DMEM/F12, containing 1×B27, EGF (20 ng/ml), bFGF (10 ng/ml), insulin (2 µg/ml), 1×NEAA, penicillin/streptomycin (100 U/ml/0.1 mg/ml) |
| H446 | 3000 | DMEM/F12, containing 1×B27, EGF (20 ng/ml), bFGF (20 ng/ml), insulin (5 µg/ml), BSA (0.4%), 1 ×NEAA, penicillin/streptomycin (100 U/ml/0.1 mg/ml) |

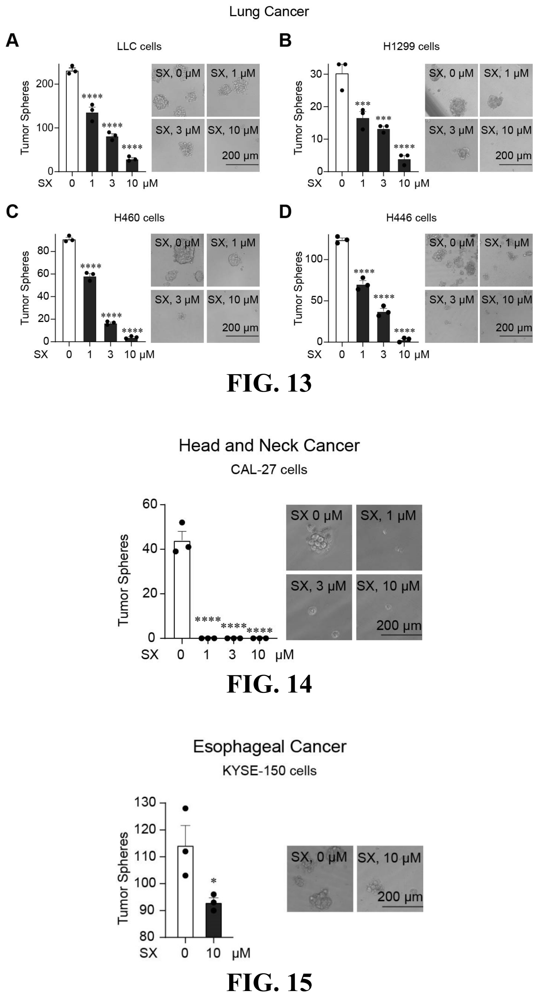

Experimental results in FIG. 13 show the number of tumor spheres from 3 independent experiments and representative tumor sphere photos. Using a One-way ANOVA analysis method and Fisher's LSD test as a post test, statistical differences in the number of tumor spheres in all the groups were analyzed (**, *P*<0.01; ***, *P*<0.001; ****, *P<*0.0001)*.* The experimental results demonstrate that the salmeterol xinafoate inhibited the self-renewal capability of the lung cancer stem cells in a concentration dependent manner.

### 1.2.4 Salmeterol xinafoate inhibits tumor sphere formation capability of head and neck cancer cells

According to culture conditions in Table 4, head and neck cancer cells CAL-27 were subjected to a tumor sphere formation assay, and treated with salmeterol xinafoate (SX) at concentrations of 1 µM, 3 µM, or 10 µM, respectively. After 7 d, tumor spheres in the control group and the SX treated group were counted and photographed, respectively.

**Table 4 Culture conditions for tumor sphere formation assay of head and neck cancer cells**

| Cells | Cell count | Culture conditions |
|---|---|---|
| CAL-27 | 1000 | DMEM/F12, containing 1×B27, EGF (20 ng/ml), bFGF (20 ng/ml), penicillin/streptomycin (100 U/ml/0.1 mg/ml) |

Experimental results in FIG. 14 show the number of tumor spheres from 3 independent experiments and representative tumor sphere photos. Using a One-way ANOVA analysis method and Fisher's LSD test as a post test, statistical differences in the number of tumor spheres in all the groups were analyzed (****, *P<*0.0001)*.* The experimental results demonstrate that the salmeterol xinafoate inhibited the self-renewal capability of the head and neck cancer stem cells.

### 1.2.5 Salmeterol xinafoate inhibits tumor sphere formation capability of esophageal cancer cells

According to culture conditions in Table 5, esophageal cancer cells KYSE-150 were subjected to a tumor sphere formation assay, and treated with salmeterol xinafoate (SX) at a concentration of 10 µM. After 7 d, tumor spheres in the control group and the SX treated group were counted and photographed, respectively.

**Table 5 Culture conditions for tumor sphere formation assay of esophageal cancer cells**

| Cells | Cell count | Culture conditions |
|---|---|---|
| KYSE-150 | 3000 | DMEM/F12, containing 1×B27, EGF (20 ng/ml), bFGF (20 ng/ml), insulin (5 µg/ml), BSA (0.5%), 1×NEAA, penicillin/streptomycin (100 U/ml/0.1 mg/ml) |

Experimental results in FIG. 15 show the number of tumor spheres from 3 independent experiments and representative tumor sphere photos. Using a One-way ANOVA analysis method and Fisher's LSD test as a post test, statistical differences in the number of tumor spheres in all the groups were analyzed (*, *P*<0.05). The experimental results demonstrate that the salmeterol xinafoate inhibited the self-renewal capability of the esophageal cancer stem cells.

### 1.2.6 Salmeterol xinafoate inhibits tumor sphere formation capability of bladder cancer cells

According to culture conditions in Table 6, bladder cancer cells RT4 were subjected to a tumor sphere formation assay, and treated with salmeterol xinafoate (SX) at concentrations of 1 µM, 3 µM, or 10 µM, respectively. After 7 d, tumor spheres in the control group and the SX treated group were counted and photographed, respectively.

**Table 6 Culture conditions for tumor sphere formation assay of bladder cancer cells**

| Cells | Cell count | Culture conditions |
|---|---|---|
| RT4 | 3000 | DMEM/F12, containing 1×B27, EGF (20 ng/ml), bFGF (10 ng/ml), 1×NEAA, penicillin/streptomycin (100 U/ml/0.1 mg/ml) |

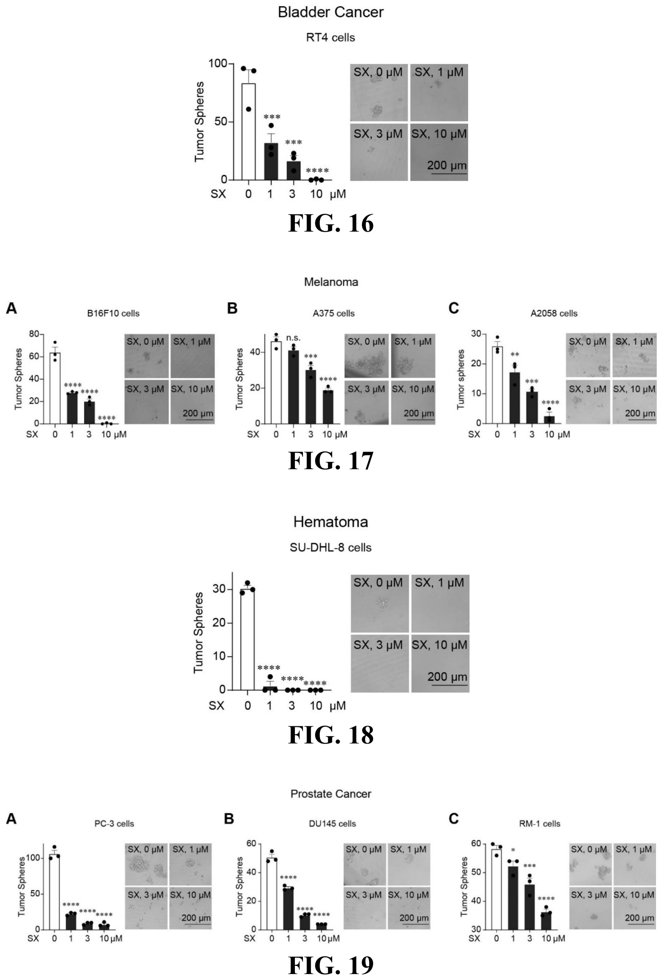

Experimental results in FIG. 16 show the number of tumor spheres from 3 independent experiments and representative tumor sphere photos. Using a One-way ANOVA analysis method and Fisher's LSD test as a post test, statistical differences in the number of tumor spheres in all the groups were analyzed (***, *P*<0.001; ****, *P*<0.0001). The experimental results demonstrate that the salmeterol xinafoate inhibited the self-renewal capability of the bladder cancer stem cells in a concentration dependent manner.

### 1.2.7 Salmeterol xinafoate inhibits tumor sphere formation capability of melanoma cells

According to culture conditions in Table 7, melanoma cells B16F10 (A), A375 (B), and A2058 (C) were subjected to tumor sphere formation assays, and treated with salmeterol xinafoate (SX) at concentrations of 1 µM, 3 µM, or 10 µM, respectively. After 7 d, tumor spheres in the control group and the SX treated group were counted and photographed, respectively.

**Table 7 Culture conditions for tumor sphere formation assay of melanoma cells**

| Cells | Cell count | Culture conditions |
|---|---|---|
| B16F10 | 3000 | DMEM/F12, containing 1×B27, EGF (20 ng/ml), bFGF (10 ng/ml), 1×NEAA, penicillin/streptomycin (100 U/ml/0.1 mg/ml) |
| A375 | 1000 | DMEM/F12, containing 1×B27, EGF (20 ng/ml), bFGF (10 ng/ml), 1×NEAA, penicillin/streptomycin (100 U/ml/0.1 mg/ml) |
| A2058 | 1000 | DMEM/F12, containing 1×B27, EGF (20 ng/ml), bFGF (10 ng/ml), 1×NEAA, penicillin/streptomycin (100 U/ml/0.1 mg/ml) |

Experimental results in FIG. 17 show the number of tumor spheres from 3 independent experiments and representative tumor sphere photos. Using a One-way ANOVA analysis method and Fisher's LSD test as a post test, statistical differences in the number of tumor spheres in all the groups were analyzed (**, *P*<0.01; ***, *P*<0.001; ****, *P*<0.0001)*.* The experimental results demonstrate that the salmeterol xinafoate inhibited the self-renewal capability of the melanoma stem cells in a concentration dependent manner.

The tumor sphere formation assays demonstrate that the salmeterol xinafoate can inhibit the self-renewal capability of cancer stem cells, and obtain consistent conclusions with the in vivo tumorigenesis experiments. This means that the in vitro tumor sphere formation assays can demonstrate that the long-acting β₂AR agonist inhibits the self-renewal capability of cancer stem cells, which reflects that the long-acting β₂AR agonist inhibits the cancer initiation and progression. Moreover, a 3R principle of experimental animals is followed, that is, based on consistent experimental conclusions, in vitro cell experiments are used as much as possible instead of in vivo animal experiments.

Therefore, this example uses the tumor sphere formation assays to demonstrate the therapeutic effects of the salmeterol xinafoate on other common cancers (hematoma, prostate cancer, liver cancer, ovarian cancer, kidney cancer, gastric cancer, pancreatic cancer, brain cancer, osteosarcoma, and cervical cancer).

### 1.2.8 Salmeterol xinafoate inhibits tumor sphere formation capability of hematoma cells

According to culture conditions in Table 8, hematoma cells SU-DHL-8 were subjected to a tumor sphere formation assay, and treated with salmeterol xinafoate (SX) at concentrations of 1 µM, 3 µM, or 10 µM, respectively. After 7 d, tumor spheres in the control group and the SX treated group were counted and photographed, respectively.

**Table 8 Culture conditions for tumor sphere formation assay of hematoma cells**

| Cells | Cell count | Culture conditions |
|---|---|---|
| SU-DHL-8 | 2000 | DMEM/F12, containing 1×B27, EGF (20 ng/ml), bFGF (20 ng/ml), insulin (5 µg/ml), 1×NEAA, penicillin/streptomycin (100 U/ml/0.1 mg/ml) |

Experimental results in FIG. 18 show the number of tumor spheres from 3 independent experiments and representative tumor sphere photos. Using a One-way ANOVA analysis method and Fisher's LSD test as a post test, statistical differences in the number of tumor spheres in all the groups were analyzed (****, *P*<0.0001)*.* The experimental results demonstrate that the salmeterol xinafoate inhibited the self-renewal capability of the hematoma stem cells in a concentration dependent manner.

### 1.2.9 Salmeterol xinafoate inhibits tumor sphere formation capability of prostate cancer cells

According to culture conditions in Table 9, prostate cancer cells PC-3 (A), DU145 (B), and RM-1 (C) were subjected to tumor sphere formation assays, and treated with salmeterol xinafoate (SX) at concentrations of 1 µM, 3 µM, or 10 µM, respectively. After 7 d, tumor spheres in the control group and the SX treated group were counted and photographed, respectively.

**Table 9 Culture conditions for tumor sphere formation assay of prostate cancer cells**

| Cells | Cell count | Culture conditions |
|---|---|---|
| PC-3 | 1000 | DMEM/F12, containing 1×B27, EGF (20 ng/ml), bFGF (20 ng/ml), insulin (3 µg/ml), 1×NEAA, penicillin/streptomycin (100 U/ml/0.1 mg/ml) |
| DU145 | 1000 | DMEM/F12, containing 1×B27, EGF (20 ng/ml), bFGF (20 ng/ml), insulin (3 µg/ml), 1×NEAA, penicillin/streptomycin (100 U/ml/0.1 mg/ml) |
| RM-1 | 1000 | DMEM/F12, containing 1×B27, EGF (20 ng/ml), bFGF (20 ng/ml), insulin (10 µg/ml), hydrocortisone (20 µg/ml), 1 ×NEAA, penicillin/streptomycin (100 U/ml/0.1 mg/ml) |

Experimental results in FIG. 19 show the number of tumor spheres from 3 independent experiments and representative tumor sphere photos. Using a One-way ANOVA analysis method and Fisher's LSD test as a post test, statistical differences in the number of tumor spheres in all the groups were analyzed (*, *P*<0.05; ***, *P*<0.001; ****, *P*<0.0001)*.* The experimental results demonstrate that the salmeterol xinafoate inhibited the self-renewal capability of the prostate cancer stem cells in a concentration dependent manner.

### 1.2.10 Salmeterol xinafoate inhibits tumor sphere formation capability of liver cancer cells

According to culture conditions in Table 10, liver cancer cells Hep G2 were subjected to a tumor sphere formation assay, and treated with salmeterol xinafoate (SX) at concentrations of 1 µM, 3 µM, or 10 µM, respectively. After 7 d, tumor spheres in the control group and the SX treated group were counted and photographed, respectively.

**Table 10 Culture conditions for tumor sphere formation assay of liver cancer cells**

| Cells | Cell count | Culture conditions |
|---|---|---|
| Hep G2 | 1000 | DMEM/F12, containing 1×B27, EGF (20 ng/ml), bFGF (20 ng/ml), 1×NEAA, penicillin/streptomycin (100 U/ml/0.1 mg/ml) |

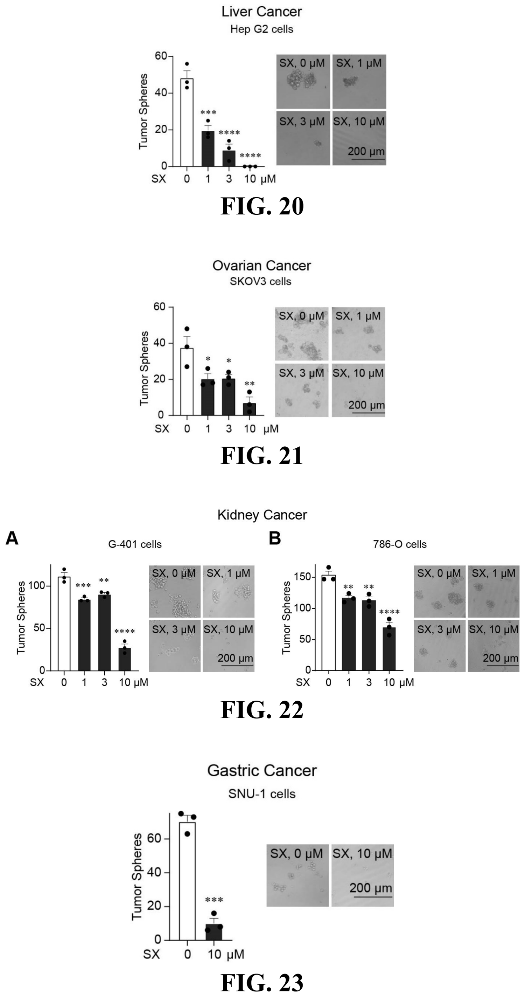

Experimental results in FIG. 20 show the number of tumor spheres from 3 independent experiments and representative tumor sphere photos. Using a One-way ANOVA analysis method and Fisher's LSD test as a post test, statistical differences in the number of tumor spheres in all the groups were analyzed (***, *P*<0.001; ****, *P*<0.0001). The experimental results demonstrate that the salmeterol xinafoate inhibited the self-renewal capability of the liver cancer stem cells in a concentration dependent manner.

### 1.2.11 Salmeterol xinafoate inhibits tumor sphere formation capability of ovarian cancer cells

According to culture conditions in Table 11, ovarian cancer cells SKOV3 were subjected to a tumor sphere formation assay, and treated with salmeterol xinafoate (SX) at concentrations of 1 µM, 3 µM, or 10 µM, respectively. After 7 d, tumor spheres in the control group and the SX treated group were counted and photographed, respectively.

**Table 11 Culture conditions for tumor sphere formation assay of ovarian cancer cells**

| Cells | Cell count | Culture conditions |
|---|---|---|
| SKOV3 | 2000 | DMEM/F12, containing EGF (20 ng/ml), bFGF (10 ng/ml), heparin (5 µg/ml), BSA (0.4%), penicillin/streptomycin (100 U/ml/0.1 mg/ml) |

Experimental results in FIG. 21 show the number of tumor spheres from 3 independent experiments and representative tumor sphere photos. Using a One-way ANOVA analysis method and Fisher's LSD test as a post test, statistical differences in the number of tumor spheres in all the groups were analyzed (*, *P*<0.05; **, *P*<0.01). The experimental results demonstrate that the salmeterol xinafoate inhibited the self-renewal capability of the ovarian cancer stem cells in a concentration dependent manner.

### 1.2.12 Salmeterol xinafoate inhibits tumor sphere formation capability of kidney cancer cells

According to culture conditions in Table 12, kidney cancer cells G-401 (A) and 786-O (B) were subjected to tumor sphere formation assays, and treated with salmeterol xinafoate (SX) at concentrations of 1 µM, 3 µM, or 10 µM, respectively. After 7 d, tumor spheres in the control group and the SX treated group were counted and photographed, respectively.

**Table 12 Culture conditions for tumor sphere formation assay of kidney cancer cells**

| Cells | Cell count | Culture conditions |
|---|---|---|
| G-401 | 1000 | DMEM/F12, containing 1×B27, EGF (20 ng/ml), bFGF (20 ng/ml), 1×NEAA, penicillin/streptomycin (100 U/ml/0.1 mg/ml) |
| 786-O | 1000 | DMEM/F12, containing 1×B27, EGF (20 ng/ml), bFGF (20 ng/ml), 1×NEAA, penicillin/streptomycin (100 U/ml/0.1 mg/ml) |

Experimental results in FIG. 22 show the number of tumor spheres from 3 independent experiments and representative tumor sphere photos. Using a One-way ANOVA analysis method and Fisher's LSD test as a post test, statistical differences in the number of tumor spheres in all the groups were analyzed (**, *P*<0.01; ***, *P*<0.001; ****, *P*<0.0001)*.* The experimental results demonstrate that the salmeterol xinafoate inhibited the self-renewal capability of the kidney cancer stem cells in a concentration dependent manner.

### 1.2.13 Salmeterol xinafoate inhibits tumor sphere formation capability of gastric cancer cells

According to culture conditions in Table 13, gastric cancer cells SNU-1 were subjected to a tumor sphere formation assay, and treated with salmeterol xinafoate (SX) at a concentration of 10 µM. After 7 d, tumor spheres in the control group and the SX treated group were counted and photographed, respectively.

**Table 13 Culture conditions for tumor sphere formation assay of gastric cancer cells**

| Cells | Cell count | Culture conditions |
|---|---|---|
| SNU-1 | 1000 | RPMI-1640, containing 1×B27, EGF (20 ng/ml), bFGF (20 ng/ml), 1×NEAA, penicillin/streptomycin (100 U/ml/0.1 mg/ml) |

Experimental results in FIG. 23 show the number of tumor spheres from 3 independent experiments and representative tumor sphere photos. Using a One-way ANOVA analysis method and Fisher's LSD test as a post test, statistical differences in the number of tumor spheres in all the groups were analyzed (***, *P*<0.001)*.* The experimental results demonstrate that the salmeterol xinafoate inhibited the self-renewal capability of the gastric cancer stem cells.

### 1.2.14 Salmeterol xinafoate inhibits tumor sphere formation capability of pancreatic cancer cells

According to culture conditions in Table 14, pancreatic cancer cells CFPAC-1 (A) and PANC-1 (B) were subjected to tumor sphere formation assays, and treated with salmeterol xinafoate (SX) at concentrations of 1 µM, 3 µM, or 10 µM, respectively. After 7 d, tumor spheres in the control group and the SX treated group were counted and photographed, respectively.

**Table 14 Culture conditions for tumor sphere formation assay of pancreatic cancer cells**

| Cells | Cell count | Culture conditions |
|---|---|---|
| CFPAC-1 | 3000 | DMEM/F12, containing 1×B27, EGF (20 ng/ml), bFGF (20 ng/ml), heparin (5 µg/ml), 1×NEAA, penicillin/streptomycin (100 U/ml/0.1 mg/ml) |
| PANC-1 | 1000 | DMEM/F12, containing 1×B27, EGF (10 ng/ml), bFGF (20 ng/ml), heparin (4 µg/ml) |

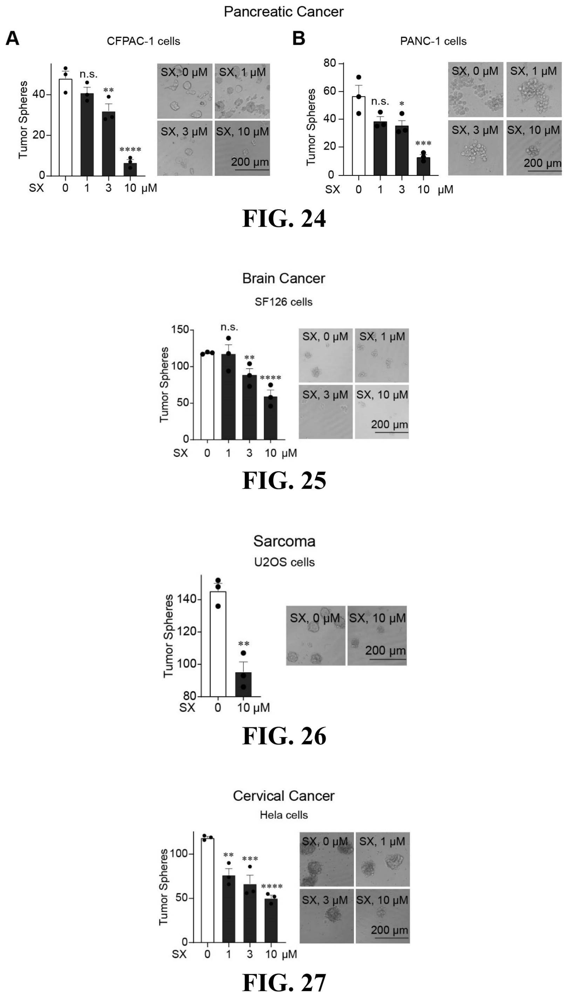

Experimental results in FIG. 24 show the number of tumor spheres from 3 independent experiments and representative tumor sphere photos. Using a One-way ANOVA analysis method and Fisher's LSD test as a post test, statistical differences in the number of tumor spheres in all the groups were analyzed (*, *P*<0.05; **, *P*<0.01; ***, *P*<0.001; ****, *P*<0.0001; n.s., no significant difference). The experimental results demonstrate that the salmeterol xinafoate inhibited the self-renewal capability of the pancreatic cancer stem cells in a concentration dependent manner.

### 1.2.15 Salmeterol xinafoate inhibits tumor sphere formation capability of brain cancer cells

According to culture conditions in Table 15, brain cancer cells SF126 were subjected to tumor sphere formation assay, and treated with salmeterol xinafoate (SX) at concentrations of 1 µM, 3 µM, or 10 µM, respectively. After 7 d, tumor spheres in the control group and the SX treated group were counted and photographed, respectively.

**Table 15 Culture conditions for tumor sphere formation assay of brain cancer cells**

| Cells | Cell count | Culture conditions |
|---|---|---|
| SF126 | 1000 | DMEM/F12, containing 1×B27, EGF (20 ng/ml), bFGF (20 ng/ml), 1 ×NEAA, penicillin/streptomycin (100 U/ml/0.1 mg/ml) |

Experimental results in FIG. 25 show the number of tumor spheres from 3 independent experiments and representative tumor sphere photos. Using a One-way ANOVA analysis method and Fisher's LSD test as a post test, statistical differences in the number of tumor spheres in all the groups were analyzed (**, *P*<0.01; ****, *P*<0.0001; n.s., no significant difference). The experimental results demonstrate that the salmeterol xinafoate inhibited the self-renewal capability of the brain cancer stem cells in a concentration dependent manner.

### 1.2.16 Salmeterol xinafoate inhibits tumor sphere formation capability of osteosarcoma cells

According to culture conditions in Table 16, osteosarcoma cells U2OS were subjected to a tumor sphere formation assay, and treated with salmeterol xinafoate (SX) at concentrations of 1 µM, 3 µM, or 10 µM, respectively. After 7 d, tumor spheres in the control group and the SX treated group were counted and photographed, respectively.

**Table 16 Culture conditions for tumor sphere formation assay of osteosarcoma cells**

| Cells | Cell count | Culture conditions |
|---|---|---|
| U2OS | 500 | DMEM/F12, containing 1×B27, EGF (20 ng/ml), bFGF (20 ng/ml), BSA (0.4%), insulin (4 µg/ml), 1 ×NEAA, penicillin/streptomycin (100 U/ml/0.1 mg/ml) |

Experimental results in FIG. 26 show the number of tumor spheres from 3 independent experiments and representative tumor sphere photos. Using a One-way ANOVA analysis method and Fisher's LSD test as a post test, statistical differences in the number of tumor spheres in all the groups were analyzed (**, *P*<0.01)*.* The experimental results demonstrate that the salmeterol xinafoate inhibited the self-renewal capability of the osteosarcoma cancer stem cells.

### 1.2.17 Salmeterol xinafoate inhibits tumor sphere formation capability of cervical cancer cells

According to culture conditions in Table 17, cervical cancer cells Hela were subjected to a tumor sphere formation assay, and treated with salmeterol xinafoate (SX) at concentrations of 1 µM, 3 µM, or 10 µM, respectively. After 7 d, tumor spheres in the control group and the SX treated group were counted and photographed, respectively.

**Table 17 Culture conditions for tumor sphere formation assay of cervical cancer cells**

| Cells | Cell count | Culture conditions |
|---|---|---|
| Hela | 1000 | DMEM/F12, containing 1×B27, EGF (20 ng/ml), bFGF (20 ng/ml), BSA (0.4%), penicillin/streptomycin (100 U/ml/0.1 mg/ml) |

Experimental results in FIG. 27 show the number of tumor spheres from 3 independent experiments and representative tumor sphere photos. Using a One-way ANOVA analysis method and Fisher's LSD test as a post test, statistical differences in the number of tumor spheres in all the groups were analyzed (**, *P*<0.01; ***, *P*<0.001; ****, *P<*0.0001)*.* The experimental results demonstrate that the salmeterol xinafoate inhibited the self-renewal capability of the cervical cancer stem cells in a concentration dependent manner.

### Example 2: Therapeutic effects of arformoterol tartrate on cancer

This example demonstrates a capability of arformoterol tartrate, that inhibits tumor sphere formation of cancer cells, in treating cancer.

According to culture conditions in Table 1-Table 17 of Example 1, breast cancer cells MDA-MB-231 (A), colorectal cancer cells SW620 (B), colorectal cancer cells CT-26 (C), lung cancer cells H460 (D), lung cancer cells H446 (E), melanoma cells B16F10 (F), melanoma cells A375 (G), head and neck cancer cells CAL-27 (H), bladder cancer cells RT4 (I), hematoma cells SU-DHL-8 (J), prostate cancer cells PC-3 (K), liver cancer cells Hep G2 (L), kidney cancer cells 786-O (M), gastric cancer cells SNU-1 (N), pancreatic cancer cells CFPAC-1 (O), pancreatic cancer cells PANC-1 (P), brain cancer cells SF126 (Q), and cervical cancer cells Hela (R) were subjected to tumor sphere formation assays, and treated with arformoterol tartrate (AT) at concentrations of 1 µM, 3 µM, or 10 µM, respectively. After 7 d, tumor spheres in the control group and the AT treated group were counted and photographed, respectively.

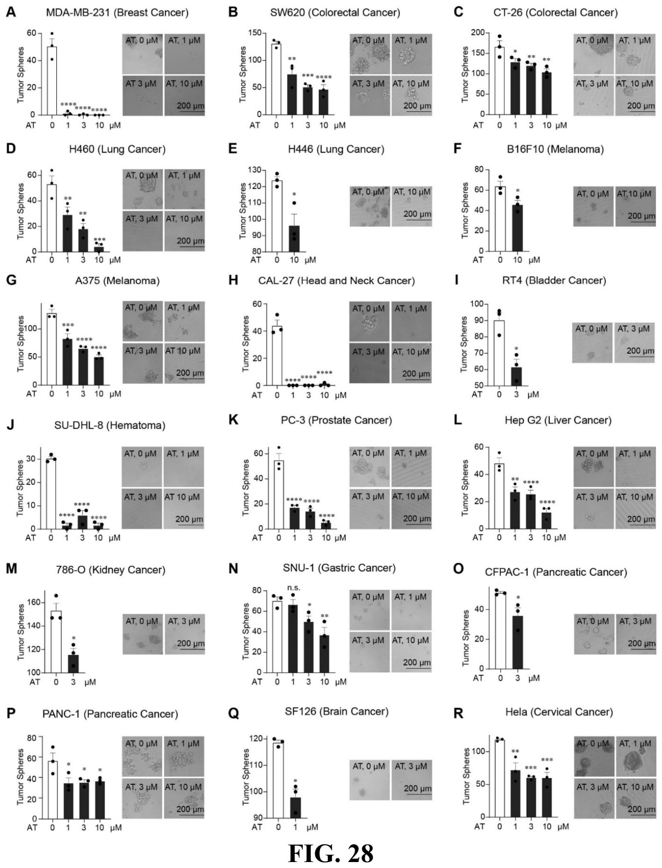

Experimental results show the number of tumor spheres from 3 independent experiments and representative tumor sphere photos. Using a One-way ANOVA analysis method and Fisher's LSD test as a post test, statistical differences in the number of tumor spheres in all the groups were analyzed (*, *P*<0.05; **, *P*<0.01; ***, *P*<0.001; ****, *P*<0.0001; n.s., no significant difference). The experimental results in FIG. 28 demonstrate that the arformoterol tartrate has therapeutic effects on a variety of types of cancer in a concentration dependent manner. The specific long-acting β₂AR agonist arformoterol tartrate has therapeutic effects on breast cancer, colorectal cancer, lung cancer, melanoma, head and neck cancer, esophageal cancer, bladder cancer, hematoma, prostate cancer, liver cancer, ovarian cancer, kidney cancer, gastric cancer, pancreatic cancer, brain cancer, osteosarcoma, and cervical cancer.

### Example 3: Therapeutic effects of bambuterol hydrochloride on cancer

This example demonstrates a capability of bambuterol hydrochloride, that inhibits tumor sphere formation of cancer cells, in treating cancer.

According to culture conditions in Table 1-Table 17 of Example 1, breast cancer cells MDA-MB-231 (A), colorectal cancer cells SW620 (B), colorectal cancer cells CT-26 (C), lung cancer cells H460 (D), lung cancer cells H446 (E), melanoma cells B16F10 (F), melanoma cells A375 (G), head and neck cancer cells CAL-27 (H), esophageal cancer cells KYSE-150 (I), bladder cancer cells RT4 (J), hematoma cells SU-DHL-8 (K), prostate cancer cells PC-3 (L), liver cancer cells Hep G2 (M), ovarian cancer cells SKOV3 (N), kidney cancer cells 786-O (O), pancreatic cancer cells CFPAC-1 (P), pancreatic cancer cells PANC-1 (Q), brain cancer cells SF126 (R), osteosarcoma cells U2OS (S), and cervical cancer cells Hela (T) were subjected to tumor sphere formation assays, and treated with bambuterol hydrochloride (BH) at concentrations of 1 µM, 3 µM, or 10 µM, respectively. After 7 d, tumor spheres in the control group and the BH treated group were counted and photographed, respectively.

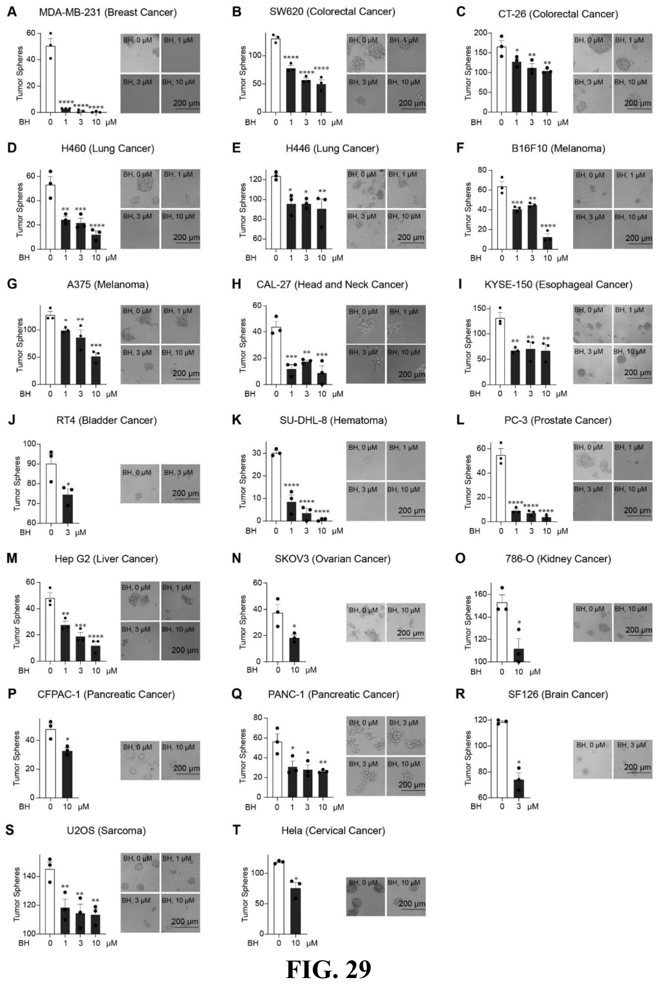

Experimental results show the number of tumor spheres from 3 independent experiments and representative tumor sphere photos. Using a One-way ANOVA analysis method and Fisher's LSD test as a post test, statistical differences in the number of tumor spheres in all the groups were analyzed (*, *P*<0.05; **, *P*<0.01; ***, *P*<0.001; ****, *P*<0.0001). The experimental results in FIG. 29 demonstrate that the bambuterol hydrochloride has therapeutic effects on a variety of types of cancer in a concentration dependent manner. Demonstrated by the tumor sphere formation assays of cancer cells, the specific long-acting β₂AR agonist bambuterol hydrochloride has therapeutic effects on breast cancer, colorectal cancer, lung cancer, melanoma, head and neck cancer, esophageal cancer, bladder cancer, hematoma, prostate cancer, liver cancer, ovarian cancer, kidney cancer, gastric cancer, pancreatic cancer, brain cancer, osteosarcoma, and cervical cancer.

### Example 4: Therapeutic effects of clenbuterol hydrochloride on cancer

This example demonstrates a capability of clenbuterol hydrochloride, that inhibits tumor sphere formation of cancer cells, in treating cancer.

According to culture conditions in Table 1-Table 17 of Example 1, breast cancer cells MDA-MB-231 (A), colorectal cancer cells SW620 (B), colorectal cancer cells CT-26 (C), lung cancer cells H460 (D), lung cancer cells H446 (E), melanoma cells B16F10 (F), melanoma cells A375 (G), head and neck cancer cells CAL-27 (H), esophageal cancer cells KYSE-150 (I), bladder cancer cells RT4 (J), hematoma cells SU-DHL-8 (K), prostate cancer cells PC-3 (L), liver cancer cells Hep G2 (M), kidney cancer cells 786-O (N), pancreatic cancer cells CFPAC-1 (O), pancreatic cancer cells PANC-1 (P), and cervical cancer cells Hela (Q) were subjected to tumor sphere formation assays, and treated with clenbuterol hydrochloride (CH) at concentrations of 1 µM, 3 µM, or 10 µM, respectively. After 7 d, tumor spheres in the control group and the CH treated group were counted and photographed, respectively.

Experimental results show the number of tumor spheres from 3 independent experiments and representative tumor sphere photos. Using a One-way ANOVA analysis method and Fisher's LSD test as a post test, statistical differences in the number of tumor spheres in all the groups were analyzed (*, *P*<0.05; **, *P<*0.01; ***, *P<*0.001; ****, *P<*0.0001)*.* The experimental results in FIG. 30 demonstrate that the clenbuterol hydrochloride has therapeutic effects on a variety of types of cancer in a concentration dependent manner. Demonstrated by the tumor sphere formation assays of cancer cells, the specific long-acting β₂AR agonist clenbuterol hydrochloride has therapeutic effects on breast cancer, colorectal cancer, lung cancer, melanoma, head and neck cancer, esophageal cancer, bladder cancer, hematoma, prostate cancer, liver cancer, ovarian cancer, kidney cancer, gastric cancer, pancreatic cancer, brain cancer, osteosarcoma, and cervical cancer.

### Example 5: Therapeutic effects of formoterol hemifumarate on cancer

This example demonstrates a capability of formoterol hemifumarate, that inhibits tumor sphere formation of cancer cells, in treating cancer.

According to culture conditions in Table 1-Table 17 of Example 1, breast cancer cells MDA-MB-231 (A), colorectal cancer cells SW620 (B), colorectal cancer cells CT-26 (C), lung cancer cells H460 (D), lung cancer cells H446 (E), melanoma cells B16F10 (F), melanoma cells A375 (G), head and neck cancer cells CAL-27 (H), esophageal cancer cells KYSE-150 (I), bladder cancer cells RT4 (J), hematoma cells SU-DHL-8 (K), prostate cancer cells PC-3 (L), liver cancer cells Hep G2 (M), kidney cancer cells 786-O (N), pancreatic cancer cells CFPAC-1 (O), pancreatic cancer cells PANC-1 (P), and cervical cancer cells Hela (Q) were subjected to tumor sphere formation assays, and treated with formoterol hemifumarate (FH) at concentrations of 1 µM, 3 µM, or 10 µM, respectively. After 7 d, tumor spheres in the control group and the FH treated group were counted and photographed, respectively.

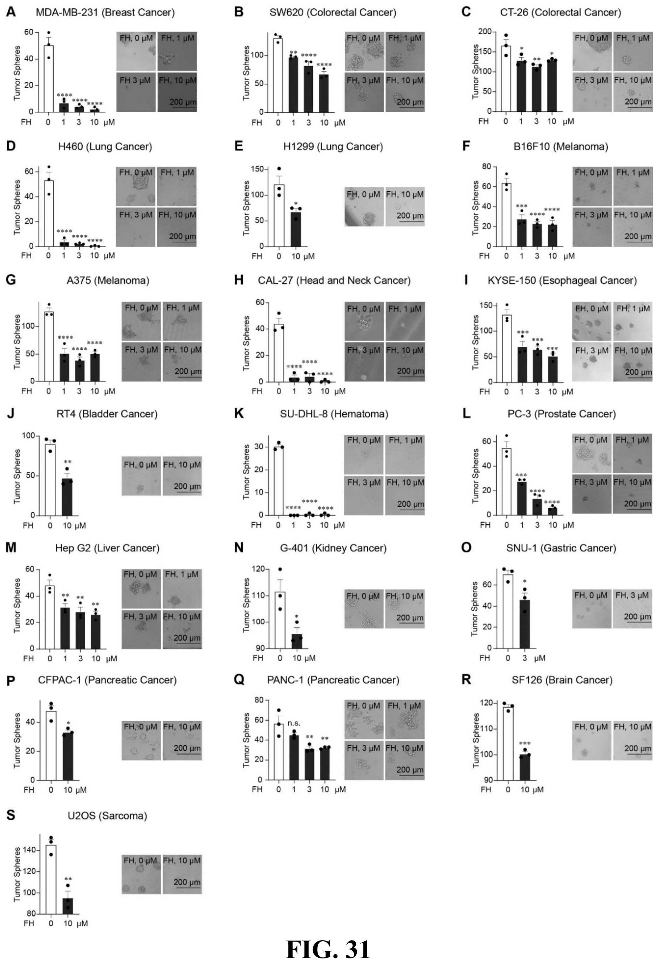

Experimental results show the number of tumor spheres from 3 independent experiments and representative tumor sphere photos. Using a One-way ANOVA analysis method and Fisher's LSD test as a post test, statistical differences in the number of tumor spheres in all the groups were analyzed (*, *P*<0.05; **, *P*<0.01; ***, *P*<0.001; ****, *P*<0.0001)*.* The experimental results in FIG. 31 demonstrate that the formoterol hemifumarate has therapeutic effects on a variety of types of cancer in a concentration dependent manner. Demonstrated by the tumor sphere formation assays of cancer cells, the specific long-acting β₂AR agonist formoterol hemifumarate has therapeutic effects on breast cancer, colorectal cancer, lung cancer, melanoma, head and neck cancer, esophageal cancer, bladder cancer, hematoma, prostate cancer, liver cancer, ovarian cancer, kidney cancer, gastric cancer, pancreatic cancer, brain cancer, osteosarcoma, and cervical cancer.

### Example 6: Therapeutic effects of indacaterol maleate on cancer

This example demonstrates a capability of indacaterol maleate, that inhibits tumor sphere formation of cancer cells, in treating cancer.

According to culture conditions in Table 1-Table 17 of Example 1, breast cancer cells MDA-MB-231 (A), colorectal cancer cells SW620 (B), colorectal cancer cells CT-26 (C), lung cancer cells H460 (D), lung cancer cells H1299 (E), lung cancer cells LLC (F), lung cancer cells H446 (G), melanoma cells B16F10 (H), melanoma cells A375 (I), head and neck cancer cells CAL-27 (J), esophageal cancer cells KYSE-150 (K), bladder cancer cells RT4 (L), hematoma cells SU-DHL-8 (M), prostate cancer cells PC-3 (N), liver cancer cells Hep G2 (O), kidney cancer cells G-401 (P), gastric cancer cells SNU-1 (Q), pancreatic cancer cells CFPAC-1 (R), pancreatic cancer cells PANC-1 (S), brain cancer cells SF126 (T), osteosarcoma cells U2OS (U), and cervical cancer cells Hela (V) were subjected to tumor sphere formation assays, and treated with indacaterol maleate (IM) at concentrations of 1 µM, 3 µM, or 10 µM, respectively. After 7 d, tumor spheres in the control group and the IM treated group were counted and photographed, respectively.

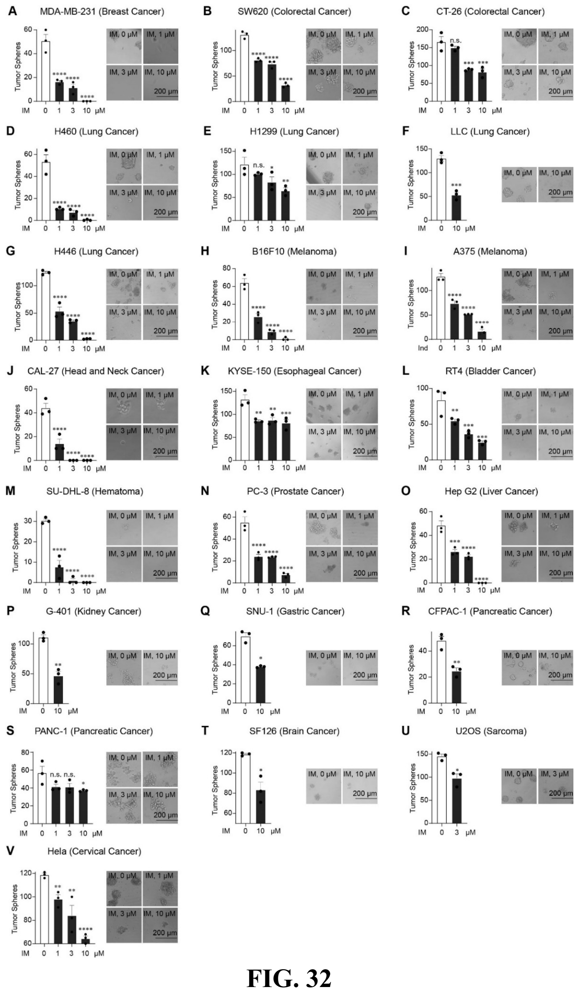

Experimental results show the number of tumor spheres from 3 independent experiments and representative tumor sphere photos. Using a One-way ANOVA analysis method and Fisher's LSD test as a post test, statistical differences in the number of tumor spheres in all the groups were analyzed (*, *P*<0.05; **, *P<*0.01; ***, *P<*0.001; ****, *P*<0.0001; n.s., no significant difference). The experimental results in FIG. 32 demonstrate that the indacaterol maleate has therapeutic effects on a variety of types of cancer in a concentration dependent manner. Demonstrated by the tumor sphere formation assays of cancer cells, the specific long-acting β₂AR agonist indacaterol maleate has therapeutic effects on breast cancer, colorectal cancer, lung cancer, melanoma, head and neck cancer, esophageal cancer, bladder cancer, hematoma, prostate cancer, liver cancer, ovarian cancer, kidney cancer, gastric cancer, pancreatic cancer, brain cancer, osteosarcoma, and cervical cancer.

### Example 7: Therapeutic effects of olodaterol hydrochloride on cancer

This example demonstrates a capability of olodaterol hydrochloride, that inhibits tumor sphere formation of cancer cells, in treating cancer.

According to culture conditions in Table 1-Table 17 of Example 1, breast cancer cells MDA-MB-231 (A), colorectal cancer cells SW620 (B), colorectal cancer cells CT-26 (C), lung cancer cells H460 (D), lung cancer cells H446 (E), melanoma cells B16F10 (F), melanoma cells A375 (G), head and neck cancer cells CAL-27 (H), esophageal cancer cells KYSE-150 (I), bladder cancer cells RT4 (J), hematoma cells SU-DHL-8 (K), prostate cancer cells PC-3 (L), liver cancer cells Hep G2 (M), ovarian cancer cells SKOV3 (N), kidney cancer cells G-401 (O), pancreatic cancer cells CFPAC-1 (P), pancreatic cancer cells PANC-1 (Q), brain cancer cells SF126 (R), osteosarcoma cells U2OS (S), and cervical cancer cells Hela (T) were subjected to tumor sphere formation assays, and treated with olodaterol hydrochloride (OH) at concentrations of 1 µM, 3 µM, or 10 µM, respectively. After 7 d, tumor spheres in the control group and the OH treated group were counted and photographed, respectively.

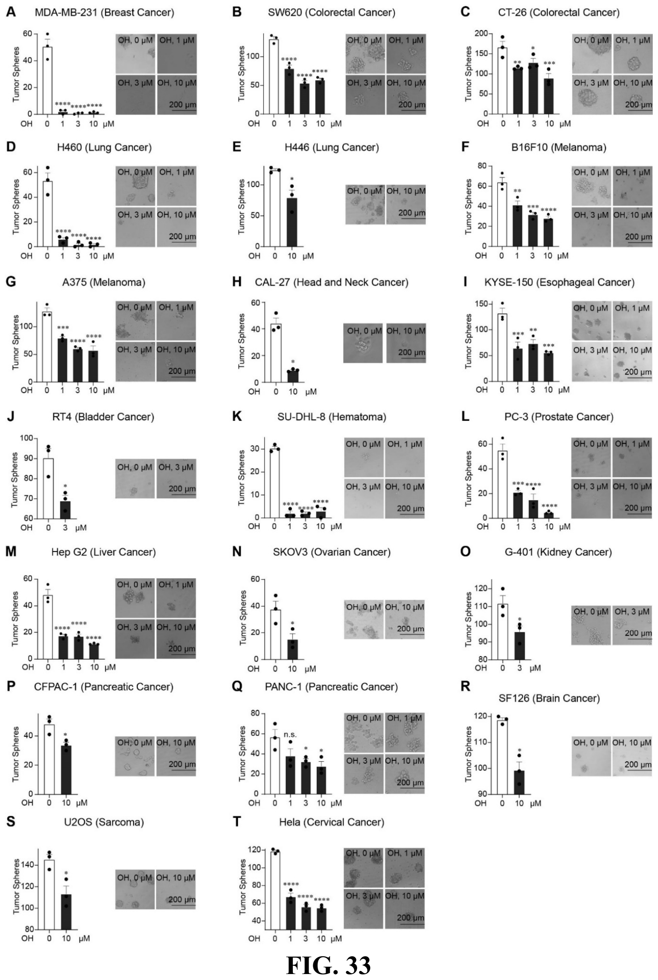

Experimental results show the number of tumor spheres from 3 independent experiments and representative tumor sphere photos. Using a One-way ANOVA analysis method and Fisher's LSD test as a post test, statistical differences in the number of tumor spheres in all the groups were analyzed (*, *P*<0.05; **, *P<*0.01; ***, *P<*0.001; ****, *P<*0.0001)*.* The experimental results in FIG. 33 demonstrate that the olodaterol hydrochloride has therapeutic effects on a variety of types of cancer in a concentration dependent manner. Demonstrated by the tumor sphere formation assays of cancer cells, the specific long-acting β₂AR agonist olodaterol hydrochloride has therapeutic effects on breast cancer, colorectal cancer, lung cancer, melanoma, head and neck cancer, esophageal cancer, bladder cancer, hematoma, prostate cancer, liver cancer, ovarian cancer, kidney cancer, gastric cancer, pancreatic cancer, brain cancer, osteosarcoma, and cervical cancer.

### Example 8: Therapeutic effects of tulobuterol hydrochloride on cancer

This example demonstrates a capability of tulobuterol hydrochloride, that inhibits tumor sphere formation of cancer cells, in treating cancer.

According to culture conditions in Table 1-Table 17 of Example 1, breast cancer cells MDA-MB-231 (A), colorectal cancer cells SW620 (B), colorectal cancer cells CT-26 (C), lung cancer cells H460 (D), lung cancer cells H1299 (E), lung cancer cells LLC (F), lung cancer cells H446 (G), melanoma cells B16F10 (H), melanoma cells A375 (I), head and neck cancer cells CAL-27 (J), esophageal cancer cells KYSE-150 (K), bladder cancer cells RT4 (L), hematoma cells SU-DHL-8 (M), prostate cancer cells PC-3 (N), liver cancer cells Hep G2 (O), kidney cancer cells G-401 (P), kidney cancer cells 786-O (Q), gastric cancer cells SNU-1 (R), pancreatic cancer cells CFPAC-1 (S), brain cancer cells SF126 (T), and cervical cancer cells Hela (U) were subjected to tumor sphere formation assays, and treated with tulobuterol hydrochloride (TH) at concentrations of 1 µM, 3 µM, or 10 µM, respectively. After 7 d, tumor spheres in the control group and the TH treated group were counted and photographed, respectively.

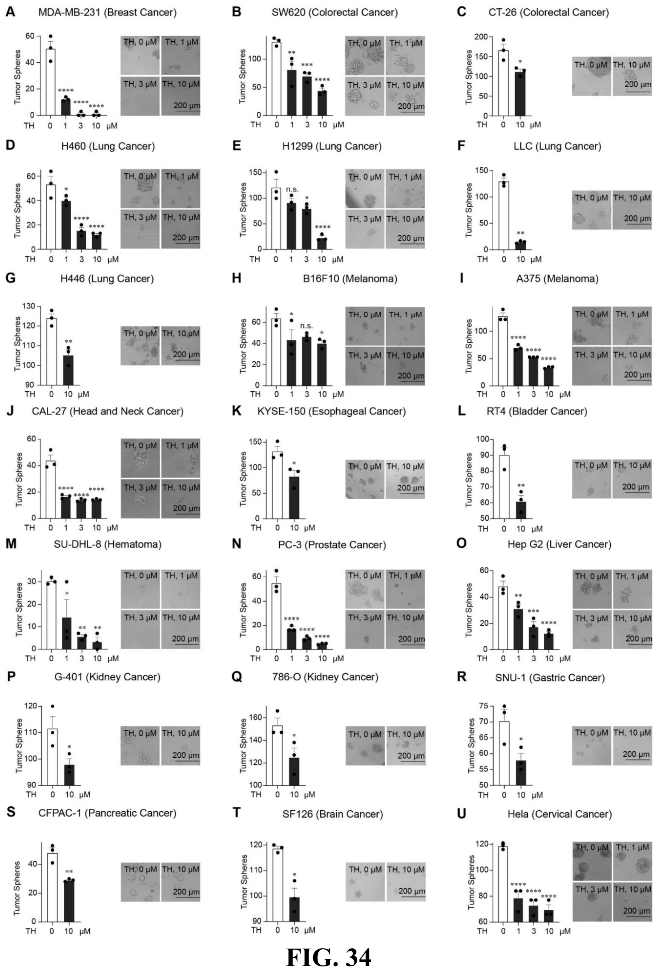

Experimental results show the number of tumor spheres from 3 independent experiments and representative tumor sphere photos. Using a One-way ANOVA analysis method and Fisher's LSD test as a post test, statistical differences in the number of tumor spheres in all the groups were analyzed (*, *P<*0.05; **, *P<*0.01; ***, *P<*0.001; ****, *P*<0.0001; n.s., no significant difference). The experimental results in FIG. 34 demonstrate that the tulobuterol hydrochloride has therapeutic effects on a variety of types of cancer in a concentration dependent manner. Demonstrated by the tumor sphere formation assays of cancer cells, the specific long-acting β₂AR agonist tulobuterol hydrochloride has therapeutic effects on breast cancer, colorectal cancer, lung cancer, melanoma, head and neck cancer, esophageal cancer, bladder cancer, hematoma, prostate cancer, liver cancer, ovarian cancer, kidney cancer, gastric cancer, pancreatic cancer, brain cancer, osteosarcoma, and cervical cancer.

### Example 9: Therapeutic effects of vilanterol trifenate on cancer

This example demonstrates a capability of vilanterol trifenate, that inhibits tumor sphere formation of cancer cells, in treating cancer.

According to culture conditions in Table 1-Table 17 of Example 1, breast cancer cells MDA-MB-231 (A), colorectal cancer cells SW620 (B), colorectal cancer cells CT-26 (C), lung cancer cells H460 (D), lung cancer cells H1299 (E), lung cancer cells LLC (F), lung cancer cells H446 (G), melanoma cells B16F10 (H), melanoma cells A375 (I), head and neck cancer cells CAL-27 (J), esophageal cancer cells KYSE-150 (K), bladder cancer cells RT4 (L), hematoma cells SU-DHL-8 (M), prostate cancer cells PC-3 (N), liver cancer cells Hep G2 (O), kidney cancer cells G-401 (P), kidney cancer cells 786-O (Q), gastric cancer cells SNU-1 (R), pancreatic cancer cells CFPAC-1 (S), pancreatic cancer cells PANC-1 (T), brain cancer cells SF126 (U), and cervical cancer cells Hela (V) were subjected to tumor sphere formation assays, and treated with vilanterol trifenate (VT) at concentrations of 1 µM, 3 µM, or 10 µM, respectively. After 7 d, tumor spheres in the control group and the VT treated group were counted and photographed, respectively.

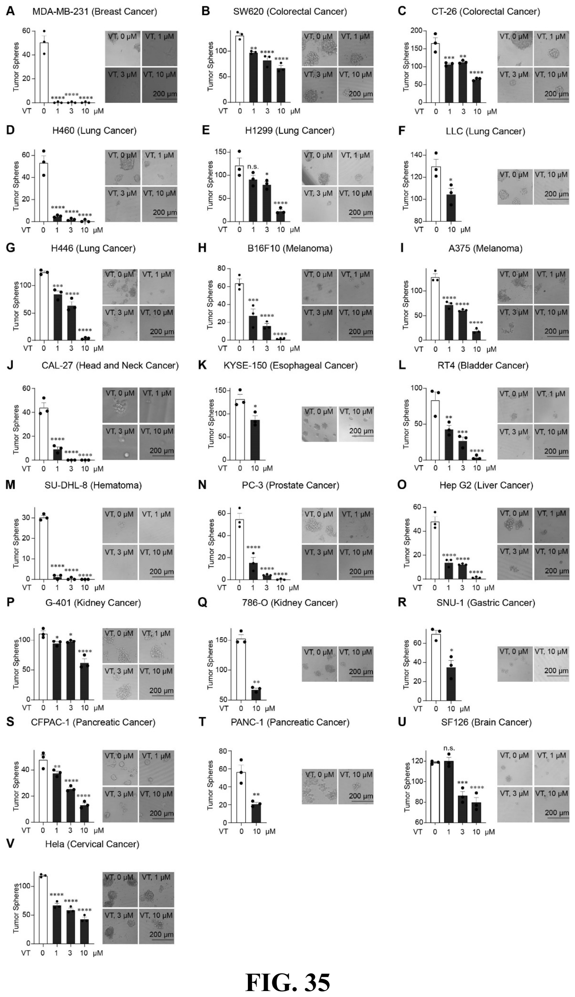

Experimental results show the number of tumor spheres from 3 independent experiments and representative tumor sphere photos. Using a One-way ANOVA analysis method and Fisher's LSD test as a post test, statistical differences in the number of tumor spheres in all the groups were analyzed (*, *P*<0.05; **, *P<*0.01; ***, *P<*0.001; ****, *P*<0.0001; n.s., no significant difference). The experimental results in FIG. 35 demonstrate that the vilanterol trifenate has therapeutic effects on a variety of types of cancer in a concentration dependent manner. Demonstrated by the tumor sphere formation assays of cancer cells, the specific long-acting β₂AR agonist vilanterol trifenate has therapeutic effects on breast cancer, colorectal cancer, lung cancer, melanoma, head and neck cancer, esophageal cancer, bladder cancer, hematoma, prostate cancer, liver cancer, ovarian cancer, kidney cancer, gastric cancer, pancreatic cancer, brain cancer, osteosarcoma, and cervical cancer.

Based on the experimental results of Examples 1-9, the examples of the present invention demonstrate that, the 9 long-acting β2AR agonists, including arformoterol tartrate, bambuterol hydrochloride, clenbuterol hydrochloride, formoterol hemifumarate, indacaterol maleate, olodaterol hydrochloride, salmeterol xinafoate, tulobuterol hydrochloride, and vilanterol trifenate have therapeutic effects on 17 cancers including breast cancer, colorectal cancer, lung cancer, melanoma, head and neck cancer, esophageal cancer, bladder cancer, hematoma, prostate cancer, liver cancer, ovarian cancer, kidney cancer, gastric cancer, pancreatic cancer, brain cancer, osteosarcoma, and cervical cancer.

Based on the experimental results of the above examples of the present invention, the following conclusions may be drawn:
1. The specific long-acting β₂AR agonists can inhibit the initiation and growth of cancer: The present invention demonstrates by orthotopic or subcutaneous tumorigenesis experiments in mice that use of a specific long-acting β₂AR agonist alone at a medicament concentration of 10 µM can significantly inhibit the initiation and progression of breast cancer, colorectal cancer, lung cancer, head and neck cancer, esophageal cancer, bladder cancer, melanoma, and other cancers.
2. The specific long-acting β₂AR agonists can inhibit the function of cancer stem cells:
   Cancer stem cells have self-renewal capability, which determines the initiation and progression of cancers. As a gold standard for detecting the self-renewal capability of cancer stem cells in vitro, a tumor sphere formation assay may detect individual cancer stem cells that continuously self-renew in a conditioned medium and form tumor spheres consisting of multiple cells.

Therefore, the present invention demonstrates by the tumor sphere formation assays of cancer cells that, use of any of the 9 specific long-acting β₂AR agonists alone, including arformoterol tartrate, bambuterol hydrochloride, clenbuterol hydrochloride, formoterol hemifumarate, indacaterol maleate, olodaterol hydrochloride, salmeterol xinafoate, tulobuterol hydrochloride, and vilanterol trifenate, can significantly inhibit the self-renewal capability of cancer stem cells of 17 cancers including breast cancer, colorectal cancer, lung cancer, head and neck cancer, esophageal cancer, bladder cancer, melanoma, hematoma, prostate cancer, liver cancer, ovarian cancer, kidney cancer, gastric cancer, pancreatic cancer, brain cancer, osteosarcoma, and cervical cancer.

Therefore, consistent conclusions were obtained from both in vitro tumor sphere formation assays and in vivo tumorigenesis experiments. The conclusions 1 and 2 demonstrate that the specific long-acting β₂AR agonists can exert a function of inhibiting cancer initiation and progression by targeting and inhibiting the self-renewal capability of cancer stem cells.
3. The β₂AR can serve as a single functional molecular target specifically expressed on the surface of a cancer stem cell membrane, and the specific long-acting β₂AR agonists are small molecule targeted medicaments to the single functional molecular target.

The β₂AR is a member of the G-protein coupled receptor superfamily, has a classic seven-transmembrane structure, and is expressed in a variety of cancers of humans and mice. The specific long-acting β₂AR agonist targets the β₂AR on the surface of a cancer cell membrane as the single functional molecular target, and perform targeted inhibition on the self-renewal capability of cancer stem cells to exert a function of inhibiting cancer initiation and progression. Therefore, the long-acting β₂AR agonists may be clinically used for treating cancer.

The examples of the present invention have been described in detail above in conjunction with the accompanying drawings, but the present invention is not limited to the above examples. Various changes may be made within the knowledge scope of those of ordinary skill in the relevant technical field without departing from the objectives of the present invention. In addition, the examples and features of the present invention may be combined with each other without conflict.

## Claims

1. Use of a long-acting β₂AR agonist or pharmaceutically acceptable salts thereof in preparation of medicaments for treating cancer.

2. The use according to claim 1, wherein the long-acting β₂AR agonist is a specific β₂ adrenoceptor agonist with a duration of efficacy of not shorter than 12 h.

3. The use according to claim 1 or 2, wherein the long-acting β₂AR agonist comprises at least one of arformoterol, bambuterol, clenbuterol, formoterol, indacaterol, olodaterol, salmeterol, tulobuterol, and vilanterol.

4. The use according to claim 3, wherein the long-acting β₂AR agonist comprises at least one of arformoterol tartrate, bambuterol hydrochloride, clenbuterol hydrochloride, formoterol hemifumarate, indacaterol maleate, olodaterol hydrochloride, salmeterol xinafoate, tulobuterol hydrochloride, and vilanterol trifenate.

5. The use according to claim 1, wherein the cancer comprises at least one of breast cancer, colorectal cancer, lung cancer, head and neck cancer, esophageal cancer, bladder cancer, melanoma, hematoma, prostate cancer, liver cancer, ovarian cancer, kidney cancer, gastric cancer, pancreatic cancer, brain cancer, osteosarcoma, and cervical cancer.

6. The use according to claim 1, wherein a dosage form of the medicaments comprises aerosol, spray, emulsion, suspension, enema, paste, ointment, gel, lotion, suppository, patch, film, capsule, tablet, pill, powder, granule, tincture, injection, syrup, or solution.

7. The use according to claim 1, wherein an administration route of the medicaments comprises oral administration, sublingual administration, inhalation administration, mucosal administration, intravenous injection, arterial injection, intramuscular injection, intradermal injection, subcutaneous injection, intraperitoneal injection, rectal administration, vaginal administration, transdermal administration, or local administration.

8. The use according to claim 1, wherein the pharmaceutically acceptable salts are salts formed by the long-acting β₂AR agonist and an acid or a base; the acid comprises an inorganic acid or an organic acid; the inorganic acid is selected from hydrochloric acid, hydrobromic acid, hydroiodic acid, nitric acid, phosphoric acid, sulfuric acid, or perchloric acid; the organic acid comprises acetic acid, oxalic acid, malic acid, maleic acid, lactic acid, pyruvic acid, methanesulfonic acid, ethanesulfonic acid, p-toluenesulfonic acid, salicylic acid, tartaric acid, benzenesulfonic acid, benzoic acid, camphorsulfonic acid, citric acid, succinic acid, fumaric acid, phenylacetic acid, triphenylacetic acid, mandelic acid, gluconic acid, glutamic acid, isethionic acid, mucic acid, pamoic acid, pantothenic acid, xinafoic acid, or malonic acid; and the base is an inorganic base containing an alkaline metal cation, alkaline-earth metal cation, or ammonium cation salt.

9. The use according to claim 1, wherein the medicaments further comprise a pharmaceutically acceptable carrier.
